# EUROPEAN PATENT SPECIFICATION

(11) **EP 3 661 902 B1**
(45) Date of publication and mention of the grant of the patent: **16.07.2025**
(21) Application number: 18745978.9
(22) Date of filing: 02.08.2018
(51) Int. Cl.: C07C 37/055, C07C 37/50, C07C 37/52, C07C 39/04, C07C 39/08, C07C 39/10, C07C 51/367, C07C 59/52, C07C 41/16, C07C 43/205, C07C 45/28, C07C 49/84

(54) **METHOD FOR THE DEACYLATION AND/OR DEALKYLATION OF COMPOUNDS**
VERFAHREN ZUR DEACYLIERUNG UND/ODER DEALKYLIERUNG VON VERBINDUNGEN
PROCÉDÉ DE DÉSACYLATION ET/OU DÉSALKYLATION DE COMPOSÉS

(30) Priority: 03.08.2017 EP 17184713
(43) Date of publication of application: 10.06.2020
(73) Proprietor: Universiteit Antwerpen, 2000 Antwerpen (BE)
(72) Inventor: MAES, Bert, 2000 Antwerpen (BE); BOMON, Jeroen, 2000 Antwerpen (BE); SERGUEEV, Serguei, 2000 Antwerpen (BE); BLONDIAUX, Enguerrand, 60200 Compiègne (FR)
(74) Representative: Arnold & Siedsma
(86) International application number: PCT/EP2018/070984
(87) International publication number: WO 2019/025535

(56) References cited:
- CN-A- 102 653 506
- JOSEPH ZAKZESKI ET AL: "Lignin Solubilization and Aqueous Phase Reforming for the Production of Aromatic Chemicals and Hydrogen", CHEMSUSCHEM, vol. 4, no. 3, 21 March 2011 (2011-03-21), pages 369 - 378, XP055017173, ISSN: 1864-5631, DOI: 10.1002/cssc.201000299
- YANG LE ET AL: "Green and efficient synthesis route of catechol from guaiacol", JOURNAL OF MOLECULAR CATALYSIS A: CHEMICAL, ELSEVIER, AMSTERDAM, NL, vol. 368, 7 December 2012 (2012-12-07), pages 61 - 65, XP028971098, ISSN: 1381-1169, DOI: 10.1016/J.MOLCATA.2012.11.024
- SEIJIRO MATSUBARA ET AL: "Palladium-Catalyzed Decarboxylation and Decarbonylation under Hydrothermal Conditions:? Decarboxylative Deuteration", ORGANIC LETTERS , 14(23), 6012-6015 CODEN: ORLEF7; ISSN: 1523-7052, vol. 6, no. 12, 1 June 2004 (2004-06-01), pages 2071 - 2073, XP055479192, ISSN: 1523-7060, DOI: 10.1021/ol0492602

## Description

### FIELD OF THE INVENTION

The present invention in general relates to a method for the deacylation and/or dealkylation (both O-dealkylation as well as C-dealkylation) of compounds, more specifically of aromatic compounds. The method is characterized by contacting the compound with an acid-containing aqueous reaction mixture using high temperature and high pressure conditions.

### BACKGROUND TO THE INVENTION

Several methods for the O-dealkylation of aromatic compounds have been described. US2.100.228 for example discloses a process for the dealkylation of an arylalkylether, in particular the dealkylation of guaiacol to pyrocatechol. According to that process, the arylalkylether is heated in the presence of an aliphatic amine hydrohalide to a typical temperature of about 140-200 °C, while passing hydrohalic acid, for example HCI or HBr through the reaction mixture until the reaction is at least partially complete. Not only does the reaction make use of gaseous HCI, carciniogenic methyl chloride is produced as a side product of the demethylation reaction.

The action of BBr₃ as a reactant in the O-dealkylation of different arylmethyl ethers to provide phenols, has been disclosed first by J.F.W. McOmie, M.L. Watts, D.E. West in Tetrahedron, Volume 24, Issue 5, 1968, Pages 2289-2292. This method however presents the disadvantage of a high cost and the use of a highly toxic reactant, which is corrosive and instable in air, inconvenient to handle and generates a large amount of toxic waste.

J. Magano, M. H. Chen, J. D. Clark, T. Nussbaumer, J. Org. Chem., 2006, 71, 7103-7105 disclose a method for the demethylation of various aromatic ethers by reaction with 2-(diethylamino)ethanethiol. Although the 2-(diethylamino)ethanethiol is a stable product and nontoxic, it is expensive and gives rise to the production of a stoichiometric amount of waste.

WO 2014083426 discloses the use of ^{γ}-alumina, preferably carried on an acidic silica carrier as a catalyst in the demethylation or demethoxylation of aromatic compounds such as guaiacol. The catalyst may further include an oxide of Ag, Zr, Ni or Fe. The reaction may be carried out at a temperature of between 350 and 550 °C in the gas phase, in an inactive gas such as nitrogen or argon, or in the liquid phase for example using water as a solvent. However, besides demethylation also the demethoxylation is observed involving the formation of phenol. The high temperature renders the method impractical.

A few publications report on carbon dealkylation (C-dealkylation) of aromatic compounds, i.e. the removal of substituents bound through a C-atom of the aromatic ring.

Nimmanwudipong et al. have disclosed in Catal. Lett. (2012) 142, 151-160 that eugenol can be transformed into guaiacol by removal of the C-substituent in a thermal reaction at 300°C. Although the reaction requires the presence of a dedicated catalyst such as Pt/Al₂O₃ or zeolite HY, guaiacol yields remain low.

The microbiologic method disclosed by Rahouti et al in the Appl. Environ. Microbiol. 1989, 55(9), 2391 makes use of Paecilomyces variotii and Pestalotia palmarum microorganisms to convert ferulic acid into different compounds, and catechol and guaiacol were found in the mixture. The disadvantages of the method are the low yields achieved (about 6% for catechol and 8% for guaiacol) and difficulties to isolate these compounds which are contained in low concentration in a complex mixture, as well as the need to use special microorganisms.

The method disclosed by Fujita, Fujita, and Okabayashi, in Nippon Kagaku Zasshi (1971), 92, 865 gave 80% catechol as a major product, in a reaction where eugenol is heated with PhNH₂.HCl at 245-255 °C for 30 min. This method however presents the disadvantages that use is made of a stoichiometric reagent which generates waste, namely N-methylaniline and N-methyl-4-propylaniline, further aniline and its derivatives are carcinogenic.

The method disclosed by Koželj and Petrič in Synlett 2007 (11):1699-1702 for the deacylation of methoxyphenyl alkyl ketones produces phenol but presents several disadvantages such as the need of using (very) large amounts of expensive reagents (triflic acid) and aromatic solvents, as well as it presents a very long processing time.

Zakzeski et al., 2011 (ChemSusChem, 4 369-378) discloses a method for dealkylation of lignin and lignin model compounds using sulfuric acid under inert atmosphere. CN102653506 discloses a method for preparing pyrocatechol from lignin in a two-step process with guaiacol as an intermediate product. Yang et al., 2013 (Journal of Molecular Catalysis A: Chemical 368-369 (2013) 61-65) disclosed a method for the synthesis of catechol from guaiacol. All of these references relate to O-dealkylation of compounds, and neither of them teaches or even suggests a method for the C-dealkylation of this type of compounds.

Matsubara et al., 2004 (Organic Letters Vol 6, No 12; p 2071-2073) discloses methods for the decarboxylation and decarbonylation of compounds, and does not disclose methods for the dealkylation or deacylation of such compounds.

There is thus a need to a process for the defunctionalisation (i.e. deacylation and/or dealkylation) of aromatic compounds, which is simple and cost effective. The present invention therefore seeks to provide a simple and cost effective process for the defunctionalisation of aromatic compounds specially to produce phenol derivatives.

This objective is achieved using a method of the current invention, which is characterized by contacting the compound to be defunctionalized with an acid-containing aqueous reaction mixture using high temperature and high pressure conditions. Although Yang et al., 2013 (Journal of Molecular Catalysis A: Chemical 368-369 (2013) 61-65) discloses a method for conversion of guaiacol to catechol in high temperature water, no high pressure conditions have been disclosed or even suggested. Furthermore, Yang et al., 2013 does not disclose the C-dealkylation of compounds.

### SUMMARY OF THE INVENTION

The present invention provides a method for the dealkylation and/or deacylation of a compound of formula (I) 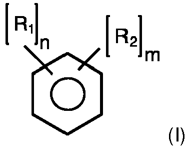 wherein:
each occurrence of R₁ is independently selected to be -OH, or -O-alkyl; wherein alkyl refers to a linear, branched or cyclic hydrocarbon group which may be saturated or contain one or more unsaturated bonds;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 1-5;
m is 1-5; and the sum of n and m is maximally 6
said method comprising the steps of:
   a) providing said compound of formula (I)
   b) if in said compound of formula (I), said R₂ contains at least one alkyl group which does not have at least one functional moiety; then:
      b1) alkylating any free -OH groups in said compound of step a); and
      b2) oxidizing in said compound of step b1); said alkyl groups which do not have said at least one functional moiety;
      wherein said at least one functional moiety is selected from the list consisting of: -OH, =O, a double bond or an amine;
   c) preparing a reaction mixture by contacting said compound of step a), or where applicable step b), with an aqueous reaction mixture containing an acid having a pKa of maximum 3.0 or a Lewis acid, under an inert gas atmosphere;
      wherein step c) is carried out at a temperature of at least 200 °C and a pressure of at least 20 bar;
   d) obtaining from step c) a phenolic compound of formula (Ia) 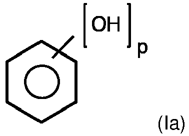
   wherein p is equal to n.

In a particular embodiment of the method from the present invention, at least one of said R₁ moieties is in ortho- or para- position with respect to at least one of said R₂ moieties.

In another particular embodiment, said acid is a mineral acid selected from the list comprising: HCI, H₃PO₄, perchloric acid, chloric acid, HI, HBr, H₂SO₄, arene sulfonic acid, alkane sulfonic acid, nitric acid or a mixture of two or more of the afore-mentioned acids, or a Lewis acid such as a salt of Fe or Cu.

In yet a further embodiment, the reaction mixture of step c) contains at least 0.05 equivalents of acid, preferably at least 0.1 equivalents with respect to the compound of formula I.

In still a further embodiment, said inert gas is selected from the list comprising: N₂, CO₂, a noble gas, such as He, Ne, Ar, a gaseous alkane such as methane, or a mixture of two or more of the aforementioned gases.

In still a further embodiment, step c) is carried out at a temperature of at least 225 °C, specifically at least 240 °C, more specifically at least 250 °C; most specifically at least 275 °C.

In a further embodiment of the present invention, step c) is carried out at a pressure of at least 30 bar, specifically at least 40 bar, more specifically at least 50 bar.

Some very specific embodiments of the present invention can be any one of the following:
- the resulting end-product of formula (Ia) is catechol and said compound of formula (I) is selected from the list comprising: caffeic acid, ferulic acid, dihydroconiferyl alcohol, propylguaiacol, and 1-(3,4-dimethoxyphenyl)propan-1-one;
- the resulting end-product of formula (Ia) is phenol and said compound of formula (I) is selected from the list comprising: coumaric acid, tyrosine (L, D, or mixture of L/D isomers).
- the resulting end-product of formula (Ia) is pyrogallol and said compound of formula (I) is selected from the list comprising: dihydrosinapyl alcohol, propylsyringol and 1-(3,4,5-trimethoxyphenyl)propan-1-one;
- the resulting end-product of formula (Ia) is phenol and said compound of formula (I) is selected from the list comprising: hydrogenated cardanol and hydrogenated cashew nut shell liquid; or
- the resulting end-product is a mixture of catechol and pyrogallol and said compound encompassing a moiety of formula (I) is a mixture selected from the list comprising: dihydroconiferyl alcohol and dihydrosinapyl alcohol; propylguaiacol and propylsyringol; 1-(3,4-dimethoxyphenyl)propan-1-one and 1-(3,4,5-trimethoxyphenyl)propan-1-one.

In a second aspect, the application provides a method not according to the claimed invention for the dealkylation of a compound encompassing a moiety of formula (II) 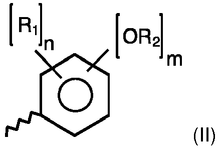 wherein: 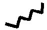 is absent or represents any carbon containing moiety;
each occurrence of R₁ is independently selected from the list comprising amines, nitro, sulphoxides, sulfonic acid, halogens, aromatic groups or alkyl groups which alkyl groups may be linear, branched or cyclic; and which alkyl groups may be saturated or containing one or more unsaturated bonds;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 0-4;
m is 1-5; and the sum of n and m is maximally 5;
said method comprising the steps of:
   a) providing said compound encompassing a moiety of formula (II)
   b) contacting said compound of step a) with an aqueous reaction mixture under an inert gas atmosphere;
      wherein said aqueous reaction mixture contains an acidic heterogenous catalyst selected from the list comprising acidic zeolite, aluminophosphate (AIPO) or silicoaluminophosphate (SAPO); and
      wherein step b) is carried out at a temperature of at least 200 °C and a pressure of at least 20 bar;
   c) obtaining from step b) a phenolic compound of formula (IIa) 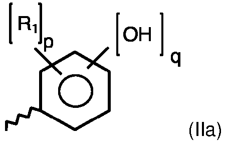 wherein p is equal to n and q is equal to m.

In a further embodiment of the second aspect (not according to the claimed invention), one or more of the following may apply:
- the inert gas is selected from the list comprising: N₂, CO₂, a noble gas, such as He, Ne, Ar, a gaseous alkane such as methane, or a mixture of two or more of the aforementioned gases;
- step b) is carried at a temperature of at least 225 °C, specifically at least 240 °C, more specifically at least 250 °C; most specifically at least 275 °C;
- step b) is carried at a pressure of at least 30 bar, specifically at least 40 bar, more specifically at least 50 bar;
- the end-product(s) of formula (IIa) are propylcatechol or propylpyrogallol or a mixture thereof and said compound(s) encompassing a moiety of formula (II) are propylguaiacol or propylsyringol or a mixture thereof; or the end-product(s) of formula (IIa) are dihydrocaffeylalcohol or 5-(3-hydroxypropyl)benzene-1,2,3-triolor a mixture thereof and said compound(s) encompassing a moiety of formula (II) are dihydroconiferylalcohol or dihydrosinapylalcohol or a mixture thereof.

### DETAILED DESCRIPTION OF THE INVENTION

As already detailed herein above, the present invention provides a method for the dealkylation and/or deacylation of compounds.

Typical reaction products that may be produced by the process of this invention, depending on the nature of the starting product used include: phenol; 1,2 dihydroxybenzene (i.e. catechol); 1,3 dihydroxybenzene (i.e. resorcinol); 1,4 dihydroxybenzene (i.e. dihydroquinone); 1,2,3 trihydroxybenzene (i.e. pyrrogallol); 1,2,4 trihydroxybenzene; and combinations of any of these. The inventors have found that with the process of this invention, end product yields, typically of over 90% may be obtained.

The process of the present invention presents the advantage of showing high yields and high selectivity towards desired end products in the removal of
(i) substituents bound to the compound, through an O-moiety, i.e. ether substituents (i.e. O-dealkylation)
(ii) substituents directly bound to a carbon atom of the compound (i.e. C-dealkylation)
(iii) functional substituents comprising a carbonyl moiety (i.e. C=O) bound via a carbon atom of the said carbonyl moiety to the compound (i.e. deacylation)

The main difference with the prior art known methods, in particular, resides in the possibility of C-dealkylation of the starting compounds, hence the ability for dealkylation and/or deacylation of compounds in which at least one of the side-chains is attached through a C atom.

Thereby, the process of this invention makes use of an acid (preferably a simple strong mineral acid), as the only reagent, without requiring the use of a reactive gas atmosphere such as H₂. This is an advantage as the use of H₂ would also require the provision of stringent safety precautions in relation to flammability and explosion risk. The process of this invention presents the further advantage that the (mineral) acids do not give rise to the generation of undesired waste as a side product. Thus a low cost process is provided which does not require the use of expensive reagents or solvents, nor does it require the provision of a stringent reaction atmosphere or particular safety precautions.

More specifically, the application also provides a method not according to the claimed invention for the dealkylation and/or deacylation of compound encompassing a moiety of formula (X) 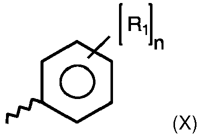 wherein: 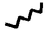 represents the attachment point of the moiety of formula (X) to the remainder of the compound; preferably it is absent or represents any carbon containing moiety;
each occurrence of R₁ is independently selected from -R₂ and -OR₂;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
wherein when -R₁ is selected to be -R₂, said alkyl group of R₂ comprises at least one functional moiety selected from the list comprising: -OH, =O, a double bond or an amine;
wherein at least one of said -R₁ is selected to be -OH, or -O-alkyl; wherein alkyl refers to a linear, branched or cyclic hydrocarbon group which may be saturated or contain one or more unsaturated bonds;
n is 2-5;
said method comprising the steps of:
   a) providing said compound encompassing a moiety of formula (X)
   b) contacting said compound of step a) with an aqueous reaction mixture under an inert gas atmosphere;

   wherein said aqueous reaction mixture contains an acidic component, selected from the list comprising: an acid having a pKa of maximum 3.0, or an acidic heterogeneous catalyst; and
   wherein step b) is carried at a temperature of at least 200 °C and a pressure of at least 20 bar.

More specifically, the present invention provides a method for the dealkylation and/or deacylation of a compound of formula (I) 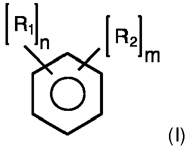 wherein:
each occurrence of R₁ is independently selected to be -OH, or -O-alkyl; wherein alkyl refers to a linear, branched or cyclic hydrocarbon group which may be saturated or contain one or more unsaturated bonds;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 1-5;
m is 1-5; and the sum of n and m is maximally 6
said method comprising the steps of:
   a) providing said compound of formula (I)
   b) if in said compound of formula (I), said R₂ contains at least one alkyl group which does not have at least one functional moiety; then:
      b1) alkylating any free -OH groups in said compound of step a); and
      b2) oxidizing in said compound of step b1); said alkyl groups which do not have said at least one functional moiety;
      wherein said at least one functional moiety is selected from the list consisting of: -OH, =O, a double bond or an amine;
   c) preparing a reaction mixture by contacting said compound of step a), or where applicable step b), with an aqueous reaction mixture containing an acid having a pKa of maximum 3.0 or a Lewis acid, under an inert gas atmosphere;
      wherein step c) is carried out at a temperature of at least 200°C and a pressure of at least 20 bar;
   d) obtaining from step c) a phenolic compound of formula (Ia) 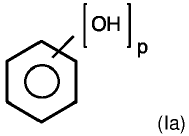
   wherein p is equal to n.

In the context of the present invention, the term "dealkylation" is meant to be the removal of a carbon chain, optionally substituted with one or more substituents, and optionally containing one or more double or triple bonds.

In the context of the present invention, the term "deacylation" is meant to be the removal of a group containing a carbonyl moiety (i.e. C=O) bound via a carbon atom of the said carbonyl moiety to the compound; said carbon chain, optionally substituted with one or more substituents, and optionally containing one or more double or triple bonds.

When describing the compounds of the invention, the terms used are to be construed in accordance with the following definitions, unless a context dictates otherwise:
The term "alkyl" by itself or as part of another substituent refers to a linear, branched or cyclic hydrocarbon group; which may be saturated or contain one or more unsaturated bonds. Generally, alkyl groups of this invention comprise from 1 to 20 carbon atoms. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. C₁-C₆ alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl.

The term "optionally substituted alkyl" refers to an alkyl group optionally substituted with one or more substituents (for example 1 to 4 substituents, for example 1, 2, 3, or 4 substituents or 1 to 2 substituents) at any available point of attachment. Non-limiting examples of such substituents include halo, hydroxyl, carbonyl, nitro, amino, oxime, imino, azido, hydrazino, cyano, aryl, heteroaryl, cycloalkyl, acyl, alkylamino, alkoxy, thiol, alkylthio, carboxylic acid, acylamino, alkyl esters, carbamate, thioamido, urea, sullfonamido and the like.

In the context of the present invention, the term "alkyl" is meant to include "alkenyl" and "alkynyl" groups which are straight-chain, cyclic, or branched-chain hydrocarbon radicals containing at least one carbon-carbon double or triple bond respectively.

The term "aromatic group" or "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. A non-limiting example of aryl or aromatic group is for example phenyl.

The aryl ring can optionally be substituted by one or more substituents. An "optionally substituted aryl" refers to an aryl having optionally one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO₂-NH₂, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO₂R^{a}, alkylthio, carboxyl, and the like, wherein R^{a} is alkyl or cycloalkyl.

The term "carboxy" or "carboxyl" or "hydroxycarbonyl" by itself or as part of another substituent refers to the group -CO₂H. Thus, a carboxyalkyl is an alkyl group as defined above having at least one substituent that is -CO₂H.

The term "Amine" is defined as an -NH₂ moiety, in which one or two hydrogen atoms are optionally replaced by an alkyl chain.

In a specific embodiment, , the application provides a method not according to the claimed invention for dealkylation and/or deacylation of compound encompassing a moiety of formula (Xb) 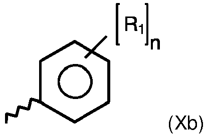 wherein:
each occurrence of R₁ is independently selected from -R₂ and -OR₂;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
wherein when -R₁ is selected to be -R₂, said alkyl group of R₂ comprises at least one functional moiety selected from the list comprising: -OH, =O, a double bond or an amine;
wherein at least one of said -R₁ is selected to be -OH, or -O-alkyl;
n is 2-5;
said method comprising the steps of:
   a) providing said compound encompassing a moiety of formula (Xb)
   b) contacting said compound of step a) with an aqueous reaction mixture under an inert gas atmosphere;
      wherein said aqueous reaction mixture contains an acidic component, selected from the list comprising: an acid having a pKa of maximum 3.0, or an acidic heterogenous catalyst; and
      wherein step b) is carried at a temperature of at least 200 °C and a pressure of at least 20 bar.

In a further embodiment, the application provides a method not according to the claimed invention for the dealkylation (C-dealkylation) of a compound encompassing a moiety of formula (XI) 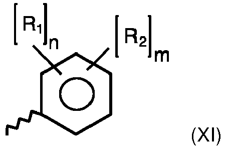 wherein: 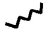 represents the attachment point of the moiety of formula (XI) to the remainder of the compound; preferably it is absent or represents any carbon containing moiety;
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
and said alkyl group of R₂ comprises at least one functional moiety selected from the list comprising: -OH, a double bond or an amine;
n is 1-4;
m is 1-4.

In a further embodiment, the present invention provides a method as defined herein, more specifically for the dealkylation (C-dealkylation) of a compound encompassing a moiety of formula (XIb) 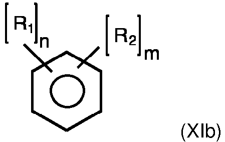 wherein:
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
and said alkyl group of R₂ comprises at least one functional moiety selected from the list comprising: -OH, a double bond or an amine;
n is 1-4;
m is 1-4.

In a further embodiment, , the application provides a method not according to the claimed invention for the deacylation of a compound encompassing a moiety of formula (XI) 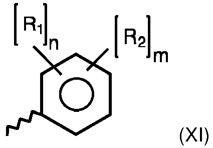 wherein: 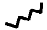 represents the attachment point of the moiety of formula (XI) to the remainder of the compound; preferably it is absent or represents any carbon containing moiety;
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
and said alkyl group of R₂ comprises at least one carbonyl moiety (i.e. C=O) bound via a carbon atom of the said carbonyl moiety to the compound;
n is 1-4;
m is 1-4.

In a further embodiment, the present invention provides a method as defined herein, more specifically for the deacylation of a compound encompassing a moiety of formula (XIb) 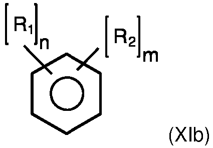 wherein:
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
and said alkyl group of R₂ comprises at least one carbonyl moiety (i.e. C=O) bound via a carbon atom of the said carbonyl moiety to the compound
n is 1-4;
m is 1-4.

In a further embodiment, the application provides a method not according to the claimed invention for the dealkylation (O-dealkylation) of a compound encompassing a moiety of formula (XII) 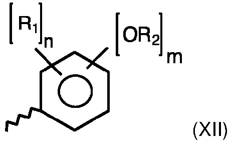 wherein: 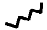 represents the attachment point of the moiety of formula (XII) to the remainder of the compound; preferably it is absent or represents any carbon containing moiety;
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 1-4;
m is 1-4.

In a further embodiment, the application provides a method not according to the claimed invention for the dealkylation (O-dealkylation) of a compound encompassing a moiety of formula (Xllb) 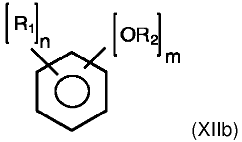 wherein:
each occurrence of R₁ is independently selected to be -OH, or -O-alkyl;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 1-4;
m is 1-4.

In yet a further embodiment and with respect to the C-dealkylation methods as defined herein, preferably, at least one of said R₁ moieties is in ortho- or para- position with respect to at least one of said R₂.

In yet a further embodiment of the methods of the present invention; said acid having a pKa of maximum 3.0 is selected from the list comprising: HCI, H₃PO₄, perchloric acid, chloric acid, HI, HBr, H₂SO₄, arene sulfonic acid, alkane sulfonic acid, nitric acid or a mixture of two or more of the afore-mentioned acids. In another embodiment of the methods of the present application not encompassed by the wording of the claims; said acidic heterogenous catalyst is selected from the list comprising: an acidic zeolite or similar acidic heterogeneous catalyst. Preferred acids for use in the present invention are strong BrØnsted or Lewis acids with a pKa of maximum 3.0, preferably maximum 2.5, maximum 2.0, maximum 1.5 or maximum 1.0, more preferably maximum 0, most preferably maximum -1.0, in particular maximum -2.0. Examples of acids suitable for use in the invention include mineral acids selected from the group of HCI (pKₐ = - 6.3), perchloric acid (pKa about -10), chloric acid HCIO₃ (pKₐ = -1.0), HI (pKₐ = -9.3), HBr (pKₐ = -8.7), H₂SO₄ (pKₐ₁ = -3.0), arene sulfonic acid for example p-toluenesulfonic acid (pKₐ = -2.8), alkane sulfonic acid for example methanesulfonic acid (pKₐ = -1.92), nitric acid HNO₃ (pKₐ = - 1.64), but also fluoroantimonic acid, FSO₃HSbF₅, carborane superacid, fluorosulfuric acid FSO₃H (pKₐ = -6.4) and triflic acid CF₃SO₃H (pKₐ = -5.9). The use of weak acids, i.e. acids with a pKa above 2 or 3 usually results in conversion rates which are of low or no economic interest.

The amount of acid present in the reaction mixture is preferably at least 0.05 equivalents of acid, preferably at least 0.1 equivalents with respect to the reactant of formula I or II, (or alternatively Xlb), in particular at 1M concentration of the substrate, to ensure a sufficiently high conversion and yield of the desired end product.

The process of this invention is carried out in an atmosphere which is inert with respect to the substrate, any intermediates produced in the course of the process of this invention and any reactants used, in order to minimize the risk to reaction, degradation and/or oxidation of the reaction products or intermediates. Any gas which is inert with respect to the reactions taking place in the present invention may be suitably used. Particularly preferred inert gases are selected from the group of N₂, CO₂, a noble gas, in particular He, Ne, Ar, an alkane gas in particular methane, or a mixture of two or more of the afore-mentioned gases.

In order to achieve a sufficient conversion, the process of this invention is carried out at a temperature of at least 200 °C, preferably at least 225 °C, more preferably at least 240 °C, most preferably at least 250 °C, in particular at least 275 °C. Below a temperature of 200 °C conversion risks to be insufficient. Preferably the reaction is carried out at a temperature of about 250 °C, as this results in an optimum conversion - economic feasibility ratio.

The reaction is carried at a pressure of at least 20 bar, preferably at least 30 bar, more preferably at least 40 bar, most preferably about 50 bar. The elevated pressure has the effect that the risk to unwanted decomposition of the reactants and/or reaction products and boiling of the aqueous phase can be reduced to a minimum.

The process of this invention is preferably carried out in aqueous reaction mixture. The aqueous reaction mixture may exclusively comprise water as the solvent for the reaction, as this permits minimizing the risk to the occurrence of unwanted side reactions, and this is preferred. The solvent may however also contain one or more organic solvents conventionally used in the reaction of organic compounds, for example an alcohol, for example methanol, ethanol or butanol, dimethylcarbonate, DMSO, DMF or a mixture of two or more of the afore-mentioned solvents. The solvent is preferably selected such that it does not give rise to the formation of unwanted side products, or that it does not show any unwanted reaction with the reactants used. In case the aqueous reaction mixture contains an organic solvent, the volume proportion of the organic solvent will usually be not more than 50 vol. %, preferably not more than 40 or 25 vol. % with respect to the total volume of solvent used, to minimize the risk to the formation of unwanted byproducts caused by the reaction of the solvent with the reaction products or reactants.

When subjecting compounds of the invention as described above to the process of this invention, any substituents bound to the compound through a O moiety will be converted into an OH moiety (i.e. O-dealkylation) and any functional group bound to the compound through a C-C bond, for example an alkyl group (i.e. C-dealkylation) or an aldehyde group (i.e. deacylation) bound to the benzene ring, will be removed as well and be converted into a H moiety.

Compounds suitable for use within the methods of the invention may be obtained from the decomposition of biomass, in particular the decomposition of lignin. For example the lignin portion of biomass (in particular, wood) contains aromatic units which may be ideal precursors for phenols. Typical decomposition products include, without being limited hereto, molecules similar to:

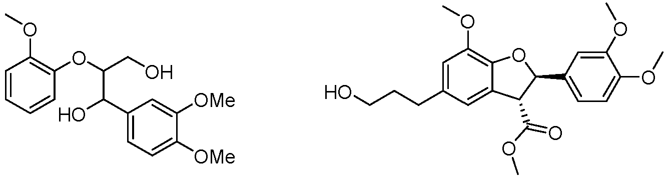

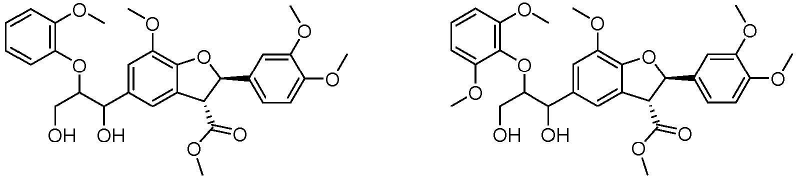

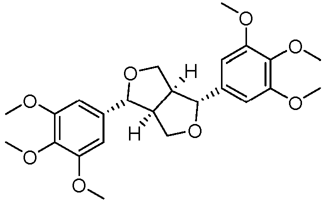

The major problem presented by biomass as a raw product is that the treatment of biomass (wood, lignin, agricultural waste etc.) typically produces aromatic derivatives with multiple additional substituents and mixtures of various aromatic derivatives which contain a wide variety of derivatives of phenol having a wide variety of substituents. The reaction product of lignin treatment is sometimes referred to as "lignin oil". In order to produce phenols from such complex mixtures, the compounds contained therein need to be subjected to a defunctionalization reaction - with the purpose of removing substituents from the complex molecule. This removal of substituents or functional groups will allow greatly simplifying the composition of the mixtures - and lignin oil or fractions thereof may be converted into mixtures of a few phenol derivatives only. The process of this invention therefore provides an economically feasible process for producing from a mixture of complex molecules, individual chemicals, or mixtures of a limited number of chemical compounds. Typical products contained in lignin pyrolysis oil include 3-methylphenol, 2-methylphenol, 3-ethylphenol, 3-propylphenol. With the process of this invention, this type of products may be converted into phenol, yielding over 90% of the desired end product. Other decomposition products contained in lignin pyrolysis oil include guaiacol, which may be converted into catechol; 2,6-dimethoxyphenol which may be converted into 1,2,3-trihydroxybenzene.

Still further decomposition products contained in lignin pyrolysis oil include 1,2,4-trimethoxybenzene, which may be converted into 1,2,4-trihydroxybenzene.

Other applications may be related to the use of certain products available from biomass in abundant amounts via biotechnologic methods such as ferulic acid, coumaric acid, caffeic acid, vanillin, produced from the rice bran and other agricultural wastes. Further examples of compounds suitable for use in the process of this invention are disclosed in the examples below.

The present invention thus permits producing phenols from biomass. Phenols (compounds containing one or more OH group on a benzene ring - e.g. phenol, catechol, resorcinol, pyrogallol) are important chemicals for many applications (e.g. resins) and chemical intermediates for different products (e.g. catechol is the key intermediate for vanillin, important flavor). Currently, phenols are produced petrochemically. Methods for the production of phenols from biomass as provided by the present invention have not been developed to date, though it appears a promising approach and the interest for such production methods is in line with the society interest in "green" products, which originate from renewable feedstock.

In yet a further aspect, the present invention provides a method as defined herein; wherein the resulting end-product is catechol and said compound encompassing a moiety of formula (I) is selected from the list comprising: caffeic acid, ferulic acid, dihydroconiferyl alcohol, propylguaiacol, and 1-(3,4-dimethoxyphenyl)propan-1-one.

In a specific embodiment, the present invention thus provides a method as defined herein; wherein the resulting end-product is phenol and said compound encompassing a moiety of formula (I) is selected from the list comprising: coumaric acid, tyrosine (L, D, or mixture of L/D isomers).

In another specific embodiment, the present invention provides a method as defined herein; wherein the resulting end-product is pyrogallol and said compound encompassing a moiety of formula (I) is selected from the list comprising: dihydrosinapyl alcohol, propylsyringol and 1-(3,4,5-trimethoxyphenyl)propan-1-one.

In yet another embodiment, the present invention provides a method as defined herein; wherein the resulting end-product is phenol and said compound encompassing a moiety of formula (I) is selected from the list comprising: hydrogenated cardanol and hydrogenated cashew nut shell liquid.

In yet a further embodiment, the present invention provides a method as defined herein; wherein the resulting end-product is a mixture of catechol and pyrogallol and said compound encompassing a moiety of formula (I) is a mixture selected from the list comprising: dihydroconiferyl alcohol and dihydrosinapyl alcohol; propylguaiacol and propylsyringol; 1-(3,4-dimethoxyphenyl)propan-1-one and 1-(3,4,5-trimethoxyphenyl)propan-1-one.

As such, some very specific embodiments of the present invention can be any one of the following:
- the resulting end-product of formula (Ia) is catechol and said compound of formula (I) is selected from the list comprising: caffeic acid, ferulic acid, dihydroconiferyl alcohol, propylguaiacol, and 1-(3,4-dimethoxyphenyl)propan-1-one;
- the resulting end-product of formula (Ia) is phenol and said compound of formula (I) is selected from the list comprising: coumaric acid, tyrosine (L, D, or mixture of L/D isomers);
- the resulting end-product of formula (Ia) is pyrogallol and said compound of formula (I) is selected from the list comprising: dihydrosinapyl alcohol, propylsyringol and 1-(3,4,5-trimethoxyphenyl)propan-1-one;
- the resulting end-product of formula (Ia) is phenol and said compound of formula (I) is selected from the list comprising: hydrogenated cardanol and hydrogenated cashew nut shell liquid; or
- the resulting end-product is a mixture of catechol and pyrogallol and said compound encompassing a moiety of formula (I) is a mixture selected from the list comprising: dihydroconiferyl alcohol and dihydrosinapyl alcohol; propylguaiacol and propylsyringol; 1-(3,4-dimethoxyphenyl)propan-1-one and 1-(3,4,5-trimethoxyphenyl)propan-1-one.

As detailed herein, the method of the present invention includes an oxidation step where non-functionalized alkyl groups are present in the starting compounds, or alkylating step and oxidation step, where free -OH and non-functionalized alkyl groups are present in the starting compounds.

Hence, the method includes a functionalization step for saturated non-functionalized alkyl chains, such that they become suitable for deacylation using the methods of the present invention. With respect to this method, it is essential though that any free -OH groups are first alkylated, since otherwise, the functionalization step, i.e. oxidation step, does not work. For such alkylation and oxidation step, any suitable process could be used, however there are a few preferred methods for doing so, as detailed below (and in the examples part).

### Alkylation method:

An exemplary suitable method for alkylation, preferably methylation of free OH groups within the present invention follows the following reaction scheme:

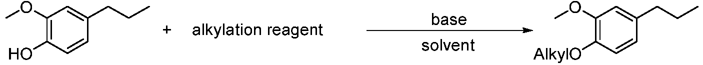

The reactions are typically carried out at a temperature ranging from 20 °C to 160 °C for a minimum of 2 h.

More specifically, suitable alkylation reagents, bases and solvents in the context of this method may be selected from the following lists:
- Alkylation reagents: dimethyl carbonate, dimethyl sulfate, tetramethylammonium chloride, methyl triflate, methyl iodide, methyl bromide, methyl chloride, diethyl carbonate, ethyl iodide, ethyl bromide, ethyl chloride
- Bases: carbonate salts such as Na₂CO₃, K₂CO₃, Cs₂CO₃; hydroxides such as NaOH, KOH, CsOH; organic bases such as trimethylamine, diethylamine or pyridine; non-nucleophilic base such as 1,8-diazabicyclo[5.4.0]undec-7-ene or phosphazenes; superbases such as amidines, guanidines or proton sponges.
- Solvents: dimethyl carbonate, DMF, acetone

### Oxidation method:

An exemplary suitable method for oxidizing the alkyl groups of R₂, within the present invention follows the following reaction scheme:

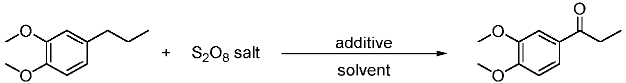

The reactions are typically carried out at a temperature of about 77 °C for a minimum of 3 h. Suitable oxidants, additives and solvents in the context of this method may be selected from the following lists:
Oxidants: S₂O₈ salts such as: K₂S₂O₈, Na₂S₂O₈, (NH₄)₂S₂O₈,
Additives: NaOAc, NaOAc.3H₂O, HOAc or Na₂CO₃
Solvents: CH₃CN/H₂O, dioxane/H₂O, H₂O, acetone/H₂O

As evident from the examples part, the use of an additive is optional, since the reaction also works without, however the yield is usually higher when a further additive is used.

Another exemplary suitable method for oxidizing the alkyl groups of R₂, within the present invention follows the following reaction scheme:

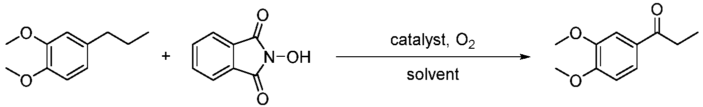

The reactions are typically carried out at a temperature of about 90 °C for a minimum of 16 h. Suitable catalysts, oxidants, additives and solvents in the context of this method may be selected from the following lists:
Catalysts: Co(OAc)₂-4H₂O, Co(acac)₂, CoCl₂·4H₂O, Co(OAc)₂·4H₂O, Co(OAc)₂·4H₂O,
Oxidant: O₂ or air or other gas mixtures containing O₂
Solvents: BuOAc, EtOAc, CH₃CN, acetic acid

Yet another exemplary suitable method for oxidizing the alkyl groups of R₂, within the present invention follows the following reaction scheme:

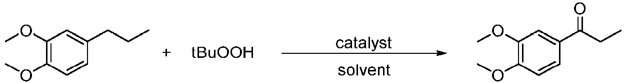

The reactions are typically carried out at a temperature ranging from 20 °C to 100 °C for a minimum of 16 h.

Suitable catalysts, oxidant, and solvents in the context of this method may be selected from the following lists:
Catalysts: CuCl, CuBr, Fe(ClO₄)₂, Fe(ClO₄)₃, FeCl₂, Fe(acac)₃, FeCl₃, FeCl₃·6H₂O
Oxidant: tBuOOH or a similar organic peroxide
Solvents: pyridine, tBuOH

Yet another exemplary suitable method for oxidizing the alkyl groups of R₂, within the present invention follows the following reaction scheme:

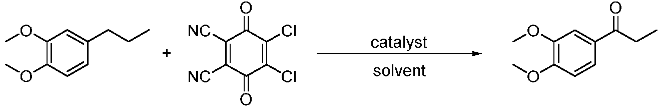

The reactions are typically carried out at a temperature of a minimum of 50 °C for a minimum of 8 h.

Suitable catalysts, oxidant, and solvents in the context of this method may be selected from the following lists:
Catalysts: formic acid, acetic acid
Oxidant: 2,3-dichloro-5,6-dicyano-1,4-benzoquinone or a similar quinone reagent
Solvents: CH₃CN/H₂O, dioxane/H₂O, H₂O, acetone/H₂O

The application also provides a method not according to the claimed invention for the dealkylation of a compound encompassing a moiety of formula (II) 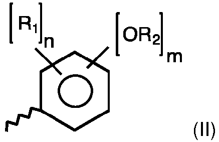 wherein: 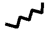 is absent or represents any carbon containing moiety;
each occurrence of R₁ is independently selected from the list comprising amines, nitro, sulphoxides, sulfonic acid, halogens, aromatic groups or alkyl groups which alkyl groups may be linear, branched or cyclic; and which alkyl groups may be saturated or containing one or more unsaturated bonds;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 0-4;
m is 1-5; and the sum of n and m is maximally 5;
said method comprising the steps of:
   a) providing said compound encompassing a moiety of formula (II)
   b) contacting said compound of step a) with an aqueous reaction mixture under an inert gas atmosphere;
      wherein said aqueous reaction mixture contains an acidic heterogenous catalyst selected from the list comprising acidic zeolite, aluminophosphate (AIPO) or silicoaluminophosphate (SAPO); and
      wherein step b) is carried out at a temperature of at least 200 °C and a pressure of at least 20 bar;
   c) obtaining from step b) a phenolic compound of formula (IIa) 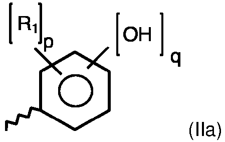 wherein p is equal to n and q is equal to m.

In a further embodiment of the second aspect of the present application not according to the claimed invention, one or more of the following may apply:
- the inert gas is selected from the list comprising: N₂, CO₂, a noble gas, such as He, Ne, Ar, a gaseous alkane such as methane, or a mixture of two or more of the aforementioned gases;
- step b) is carried at a temperature of at least 225 °C, specifically at least 240 °C, more specifically at least 250 °C; most specifically at least 275 °C;
- step b) is carried at a pressure of at least 30 bar, specifically at least 40 bar, more specifically at least 50 bar;
- the end-product(s) of formula (IIa) are propylcatechol or propylpyrogallol or a mixture thereof and said compound(s) encompassing a moiety of formula (II) are propylguaiacol or propylsyringol or a mixture thereof; or the end-product(s) of formula (IIa) are dihydrocaffeylalcohol or 5-(3-hydroxypropyl)benzene-1,2,3-triolor a mixture thereof and said compound(s) encompassing a moiety of formula (II) are dihydroconiferylalcohol or dihydrosinapylalcohol or a mixture thereof.

### Experimental procedures

### General procedure A

A 4 mL glass vial was charged with a magnetic stirring bar, the substrate for the experiment, the acid or alkaline reagent and 2 mL of the appropriate solvent or solvent mixture. The vial was closed properly with the correct cap and septum and the septum was pierced with a syringe needle. This vial was brought to the 4620 Parr reactor and the reactor was closed properly. The reactor was flushed with the appropriate gas (3 x 10 bar) and then filled with this gas (with the reported pressure). The reactor was heated to the reaction temperature and this temperature was maintained for the reported reaction time (it takes approx. 60 min to reach 250 °C). After cooling down (from 250 °C to 170 °C in the air and from 170 °C to r.t. in an ice bath), the gas was released and the reactor was opened.

After opening the reactor, the crude reaction mixture was brought to a roundbottomed flask and the vial was rinsed with H₂O (3 mL). This aqueous reaction mixture was freezed by gently rotating the flask in liq. N₂. Subsequently, vacuum was applied until all volatiles were removed. If necessary, this freeze drying step was repeated multiple times. The residue was redissolved in acetone, filtered over a silica plug and the filtrate was concentrated under reduced pressure by using a rotary evaporator. The residue was analysed with NMR and MS (APCI) or LC-MS.

### General procedure B

A 15 mL home made PTFE insert was charged with a magnetic stirring bar, the substrate for the experiment, the acid catalyst and 10 mL of the appropriate solvent or solvent mixture. This vial was left open and brought to the 4596 Parr reactor and the reactor was closed properly. The reactor was flushed with N₂ gas (3 x 10 bar) and then filled with N₂ gas (50 bar). The reactor was heated to the reaction temperature and this temperature was maintained for the reported reaction time (it takes approx. 30 min to reach 250 °C). After cooling down (from 250 °C to 170 °C in the air and from 170 °C to r.t. in an ice bath), the gas was released and the reactor was opened.

The reaction mixture was brought to a roundbottomed flask and the major part of the solvent was removed by using a rotary evaporator. Subsequently, the residue was freezed by gently rotating the flask in liq. N₂ and vacuum was applied until all volatiles were removed. If necessary, this freeze drying step was repeated multiple times. The residue was redissolved in acetone, filtered over a silica plug and the filtrate was concentrated under reduced pressure by using a rotary evaporator. The residue was analysed with NMR and MS (APCI) or LC-MS.

### General procedure C

A 50 mL round bottomed flask was charged with a magnetic stirring bar, the substrate for the experiment, the alkylation reagent, the base and the solvent. Once reaction was completed, the reaction mixture was filtered and vacuum was applied until all volatiles were removed. The crude reaction mixture was brought to a 50 mL round bottomed flask and was further charged with a magnetic stirring bar, the oxidation reagents and the appropriate solvent or solvent mixture. Once reaction was completed, the reaction mixture was diluted with water, transferred to a separation funnel and extracted with an organic solvent. Vacuum was applied until all volatiles were removed.

A 4 mL glass vial was charged with a magnetic stirring bar, the previous crude mixture, the acid or alkaline reagent and 2 mL of the appropriate solvent or solvent mixture. The vial was closed properly with the correct cap and septum and the septum was pierced with a syringe needle. This vial was brought to the 4620 Parr reactor and the reactor was closed properly. The reactor was flushed with the appropriate gas (3 x 10 bar) and then filled with this gas (with the reported pressure). The reactor was heated to the reaction temperature and this temperature was maintained for the reported reaction time (it takes approx. 60 min to reach 250°C). After cooling down (from 250 °C to 170 °C in the air and from 170 °C to r.t. in an ice bath), the gas was released and the reactor was opened.

After opening the reactor, the crude reaction mixture was brought to a round bottomed flask and the vial was rinsed with H₂O (3 mL). This aqueous reaction mixture was freezed by gently rotating the flask in liq. N₂. Subsequently, vacuum was applied until all volatiles were removed. If necessary, this freeze drying step was repeated multiple times. The residue was redissolved in acetone, filtered over a silica plug and the filtrate was concentrated under reduced pressure by using a rotary evaporator. The residue was analysed with NMR and MS (APCI) or LC-MS.

### EXAMPLES ACCORDING TO THE APPLICATION

### PART I: C-dealkylation of side chains using strong acids (combined with O-dealkylation)

### Example 1. Synthesis of catechol from eugenol (4-allyl-2-methoxyphenol) [JBO-334]

This experiment was performed according to General procedure A. Eugenol (164 mg, 1 mmol) was used as the substrate, conc. H₂SO₄ (11 µL, 0.2 mmol, 0.2 equiv.) as acidic catalyst and H₂O (2 mL) as the solvent. The reaction was performed at 250 °C for 3 h under 50 bar of N₂ pressure. Catechol was obtained in 73% isolated yield (80 mg, 0.73 mmol). ¹H NMR (400 MHz, CDCl₃) δ 6.90-6.85 (m, 2H), 6.83-6.81 (m, 2H), 5.10 (s, 2H) ppm. ¹³C NMR (101 MHz, CDCl₃): δ 143.7 (C), 121.5 (CH), 115.7 (CH) ppm. HRMS (ESI) for C₆H₇O₂ [M+H]⁺ calcd. 111.0446, found 111.0447.

### Example 2. Synthesis of catechol from isoeugenol (2-methoxy-4-prop-1-enylphenol) [JBO-307]

This experiment was performed according to General procedure A. Isoeugenol (164 mg, 1 mmol) was used as the substrate, conc. HCI (17 µL, 0.2 mmol, 0.2 equiv.) as acidic catalyst and H₂O (2 mL) as the solvent. The reaction was performed at 250 °C for 3 h under 50 bar of N₂ pressure. Catechol was obtained in 85% isolated yield (94 mg, 0.85 mmol).

### Example 3. Synthesis of catechol from ortho-eugenol (6-allyl-2-methoxyphenol) [JBO-487]

This experiment was performed according to General procedure A. Ortho-eugenol (164 mg, 1 mmol) was used as the substrate, conc. H₂SO₄ (56 µL, 1 mmol, 1 equiv.) as acidic catalyst and H₂O (2 mL) as the solvent. The reaction was performed at 250 °C for 3 h under 50 bar of N₂ pressure. Catechol was obtained in 18% NMR yield (0.18 mmol).

### Example 4. Synthesis of catechol from ferulic acid (3-(4-hydroxy-3-methoxyphenyl)prop-2-enoic acid) [JBO-684]

This experiment was performed according to General procedure A. Ferulic acid (194 mg, 1 mmol) was used as the substrate, conc. HCI (17 µL, 0.2 mmol, 0.2 equiv.) as acidic catalyst and H₂O (2 mL) as the solvent. The reaction was performed at 250 °C for 3 h under 50 bar of N₂ pressure. After cooling down, volatiles were not evaporated but mixed with DMSO-d₆ and a ¹H NMR spectrum was recorded with suppression of the H₂O signal. Catechol was obtained in 78% NMR yield (0.78 mmol) and MeOH in 92% NMR Yield (0.92 mmol).

### Example 5. Acid screening for the synthesis of catechol from ferulic acid - 0.2 equiv. H₂SO₄ [JBO-688]

This experiment was performed according to General procedure A. Ferulic acid (194 mg, 1.0 mmol) was used as the substrate, conc. H₂SO₄ (11 µL, 0.2 mmol, 0.2 equiv.) as acidic catalyst and H₂O (2 mL) as the solvent. The reaction was performed at 250 °C for 3 h under 50 bar of N₂ pressure. Catechol was obtained in 58% NMR yield (0.576 mmol), no starting material was recovered.

### Example 6. Acid screening for the synthesis of catechol from ferulic acid - 0.2 equiv. H₃PO₄ [JBO-689]

This experiment was performed according to General procedure A. Ferulic acid (194 mg, 1.0 mmol) was used as the substrate, 85% aq. H₃PO₄ (14 µL, 0.2 mmol, 0.2 equiv.) as acidic catalyst and H₂O (2 mL) as the solvent. The reaction was performed at 250 °C for 3 h under 50 bar of N₂ pressure. Catechol was obtained in 19% NMR yield (0.193 mmol), no starting material was recovered.

### Example 7. Acid screening for the synthesis of catechol from ferulic acid - 0.2 equiv. HOAc [JBO-690] - REFERENCE EXAMPLE

This experiment was performed according to General procedure A. Ferulic acid (194 mg, 1.0 mmol) was used as the substrate, HOAc (11 µL, 0.2 mmol, 0.2 equiv.) as acidic catalyst and H₂O (2 mL) as the solvent. The reaction was performed at 250 °C for 3 h under 50 bar of N₂ pressure. No catechol was obtained, no starting material was recovered.

### Example 8. Acid screening for the synthesis of catechol from ferulic acid - 0.2 equiv. H₃BO₃ [JBO-691]- REFERENCE EXAMPLE

This experiment was performed according to General procedure A. Ferulic acid (194 mg, 1.0 mmol) was used as the substrate, H₃BO₃ (12 mg, 0.2 mmol, 0.2 equiv.) as acidic catalyst and H₂O (2 mL) as the solvent. The reaction was performed at 250 °C for 3 h under 50 bar of N₂ pressure. No catechol was obtained, no starting material was recovered.

### Example 9. Acid screening for the synthesis of catechol from ferulic acid - no acid [JBO-693] - REFERENCE EXAMPLE

This experiment was performed according to General procedure A. Ferulic acid (194 mg, 1.0 mmol) was used as the substrate and H₂O (2 mL) as the solvent. The reaction was performed at 250 °C for 3 h under 50 bar of N₂ pressure. No catechol was obtained, no starting material was recovered.

### Example 10. Acid screening for the synthesis of catechol from ferulic acid - 0.01 equiv. HCI [JBO-694]

This experiment was performed according to General procedure A. Ferulic acid (194 mg, 1.0 mmol) was used as the substrate, 1 M aq. HCl (10 µL, 1.0 mmol, 0.2 equiv.) as acidic catalyst and H₂O (2 mL) as the solvent. The reaction was performed at 250 °C for 3 h under 50 bar of N₂ pressure. Catechol was obtained in 7% NMR yield (0.069 mmol), no starting material was recovered.

### Example 11. Acid screening for the synthesis of catechol from ferulic acid - 0.1 equiv. HCl [JBO-695]

This experiment was performed according to General procedure A. Ferulic acid (194 mg, 1.0 mmol) was used as the substrate, conc. HCI (8.3 µL, 0.1 mmol, 0.1 equiv.) as acidic catalyst and H₂O (2 mL) as the solvent. The reaction was performed at 250 °C for 3 h under 50 bar of N₂ pressure. Catechol was obtained in 54% NMR yield (0.54 mmol), no starting material was recovered.

### Example 12. Acid screening for the synthesis of catechol from ferulic acid - 0.5 equiv. H₂SO₄ [JBO-729]

This experiment was performed according to General procedure A. Ferulic acid (194 mg, 1.0 mmol) was used as the substrate, conc. H₂SO₄ (28 µL, 0.5 mmol, 0.5 equiv.) as acidic catalyst and H₂O (2 mL) as the solvent. The reaction was performed at 250 °C for 3 h under 50 bar of N₂ pressure. Catechol was obtained in 50% NMR yield (0.497 mmol), no starting material was recovered.

### Example 13. Acid screening for the synthesis of catechol from ferulic acid - 0.2 equiv. FeCl₃[JBO-705]

This experiment was performed according to a modified General procedure A. Ferulic acid (194 mg, 1.0 mmol) was used as the substrate, FeCl₃ (32 mg, 0.2 mmol, 0.2 equiv.) as acidic catalyst and H₂O (2 mL) as the solvent. The reaction was performed at 250 °C for 3 h under 50 bar of N₂ pressure. Before the freeze drying step, NH₄Cl (s) was added until saturation. Catechol was obtained in 50% NMR yield (0.496 mmol), no starting material was recovered.

### Example 14. Acid screening for the synthesis of catechol from ferulic acid - 0.2 equiv. FeBr₃ [JBO-707]

This experiment was performed according to a modified General procedure A. Ferulic acid (194 mg, 1.0 mmol) was used as the substrate, FeBr₃ (59 mg, 0.2 mmol, 0.2 equiv.) as acidic catalyst and H₂O (2 mL) as the solvent. The reaction was performed at 250 °C for 3 h under 50 bar of N₂ pressure. Before the freeze drying step, NH₄Cl (s) was added until saturation. Catechol was obtained in 65% NMR yield (0.647 mmol), no starting material was recovered.

### Example 15. Acid screening for the synthesis of catechol from ferulic acid - 0.2 equiv. FeCl₂ [JBO-709]

This experiment was performed according to a modified General procedure A. Ferulic acid (194 mg, 1.0 mmol) was used as the substrate, FeCl₂ (25 mg, 0.2 mmol, 0.2 equiv.) as acidic catalyst and H₂O (2 mL) as the solvent. The reaction was performed at 250 °C for 3 h under 50 bar of N₂ pressure. Before the freeze drying step, NH₄Cl (s) was added until saturation. Catechol was obtained in 42% NMR yield (0.422 mmol), no starting material was recovered.

### Example 16. Acid screening for the synthesis of catechol from ferulic acid - 0.2 equiv. FeBr₂ [JBO-747]

This experiment was performed according to a modified General procedure A. Ferulic acid (194 mg, 1.0 mmol) was used as the substrate, FeBr₂ (43 mg, 0.2 mmol, 0.2 equiv.) as acidic catalyst and H₂O (2 mL) as the solvent. The reaction was performed at 250 °C for 3 h under 50 bar of N₂ pressure. Before the freeze drying step, NH₄Cl (s) was added until saturation. Catechol was obtained in 25% NMR yield (0.247 mmol), no starting material was recovered.

### Example 17. Temperature screening for the synthesis of catechol from ferulic acid - 230 °C [JBO-759]

This experiment was performed according to General procedure A. Ferulic acid (194 mg, 1.0 mmol) was used as the substrate, conc. HCI (17 µL, 0.2 mmol, 0.2 equiv.) as acidic catalyst and H₂O (2 mL) as the solvent. The reaction was performed at 230 °C for 3 h under 50 bar of N₂ pressure. Catechol was obtained in 22% NMR yield (0.226 mmol), no starting material was recovered.

### Example 18. Temperature screening for the synthesis of catechol from ferulic acid - 240 °C [JBO-760]

This experiment was performed according to General procedure A. Ferulic acid (194 mg, 1.0 mmol) was used as the substrate, conc. HCI (17 µL, 0.2 mmol, 0.2 equiv.) as acidic catalyst and H₂O (2 mL) as the solvent. The reaction was performed at 240 °C for 3 h under 50 bar of N₂ pressure. Catechol was obtained in 38% NMR yield (0.378 mmol), no starting material was recovered.

### Example 19. Temperature screening for the synthesis of catechol from ferulic acid - 275 °C [JBO-743]

This experiment was performed according to General procedure A. Ferulic acid (194 mg, 1.0 mmol) was used as the substrate, conc. HCI (17 µL, 0.2 mmol, 0.2 equiv.) as acidic catalyst and H₂O (2 mL) as the solvent. The reaction was performed at 275 °C for 3 h under 50 bar of N₂ pressure. Catechol was obtained in 29% NMR yield (0.287 mmol), no starting material was recovered.

### Example 20. Temperature and pressure screening for the synthesis of catechol from ferulic acid - 275 °C , 75 bar [JBO-753]

This experiment was performed according to General procedure A. Ferulic acid (194 mg, 1.0 mmol) was used as the substrate, conc. HCI (17 µL, 0.2 mmol, 0.2 equiv.) as acidic catalyst and H₂O (2 mL) as the solvent. The reaction was performed at 275 °C for 3 h under 75 bar of N₂ pressure. Catechol was obtained in 49% NMR yield (0.485 mmol), no starting material was recovered.

### Example 21. Solvent screening for the synthesis of catechol from ferulic acid - H₂O / EtOH (50:50) [JBO-701]

This experiment was performed according to General procedure A. Ferulic acid (194 mg, 1.0 mmol) was used as the substrate, conc. HCI (17 µL, 0.2 mmol, 0.2 equiv.) as acidic catalyst and a mixture of H₂O (1 mL) and ethanol (1 mL) as the solvent. The reaction was performed at 250 °C for 3 h under 50 bar of N₂ pressure. Catechol was obtained in 6% NMR yield (0.058 mmol), no starting material was recovered.

### Example 22. Solvent screening for the synthesis of catechol from ferulic acid - H₂O / EtOH (20:80) [JBO-702] - REFERENCE EXAMPLE

This experiment was performed according to General procedure A. Ferulic acid (194 mg, 1.0 mmol) was used as the substrate, conc. HCI (17 µL, 0.2 mmol, 0.2 equiv.) as acidic catalyst and a mixture of H₂O (0.4 mL) and ethanol (1.6 mL) as the solvent. The reaction was performed at 250 °C for 3 h under 50 bar of N₂ pressure. No catechol was obtained, no starting material was recovered.

### Example 23. Solvent screening for the synthesis of catechol from ferulic acid - H₂O / EtOH (80:20) [JBO-703]

This experiment was performed according to General procedure A. Ferulic acid (194 mg, 1.0 mmol) was used as the substrate, conc. HCI (17 µL, 0.2 mmol, 0.2 equiv.) as acidic catalyst and a mixture of H₂O (1.6 mL) and ethanol (0.4 mL) as the solvent. The reaction was performed at 250 °C for 3 h under 50 bar of N₂ pressure. Catechol was obtained in 18% NMR yield (0.18 mmol), no starting material was recovered.

### Example 24. Synthesis of phenol from para-coumaric acid [JBO-657]

This experiment was performed according to a modified General procedure A. Para-coumaric acid (82 mg, 0.5 mmol) was used as the substrate, conc. H₂SO₄ (5.6 µL, 1 mmol, 1 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d6 and ¹H NMR was performed with suppression of the H₂O signal. Phenol was obtained in 22% NMR yield (0.108 mmol).

### Example 25. Synthesis of catechol from dihydroconiferylalcohol (4-(3-hydroxy-1-propenyl)-2-methoxyphenol) [JBO-370]

This experiment was performed according to General procedure B. Dihydroconiferylalcohol (1.82 g, 10 mmol) was used as the substrate, conc. HCI (0.56 mL, 10 mmol, 1 equiv.) as acidic catalyst and H₂O (10 mL) as the solvent. The reaction was performed at 250 °C for 3 h under 50 bar of N₂ pressure. Catechol was obtained in 84% yield.

### Example 26. Synthesis of catechol from 4-(2-hydroxypropyl)-2-methoxyphenol [JBO-367]

This experiment was performed according to General procedure A. 4-(2-Hydroxypropyl)-2-methoxyphenol (91 mg, 0.5 mmol) was used as the substrate, conc. HCI (8.5 µL, 0.1 mmol, 0.2 equiv.) as acidic catalyst and H₂O (1 mL) as the solvent. The reaction was performed at 250 °C for 3 h under 50 bar of N₂ pressure. Catechol was obtained (based on MS and NMR analysis).

### Example 27. Synthesis of catechol from 4-(1-hydroxypropyl)-2-methoxyphenol [JBO-476]

This experiment was performed according to General procedure A. 4-(1-Hydroxypropyl)-2-methoxyphenol (91 mg, 0.5 mmol) was used as the substrate, conc. HCI (8.5 µL, 0.1 mmol, 0.2 equiv.) as acidic catalyst and H₂O (1 mL) as the solvent. The reaction was performed at 250 °C for 3 h under 50 bar of N₂ pressure. Catechol was obtained in 97% isolated yield (1.07 g, 9.72 mmol).

### Example 28. Synthesis of pyrogallol from dihydrosinapylalcohol (4-(3-hydroxypropyl)-2,6-dimethoxyphenol) [JBO-507]

This experiment was performed according to a modified General procedure A. Dihydrosinapylalcohol (212 mg, 1.0 mmol) was used as the substrate, FeCl₃ (32 mg, 0.2 mmol, 0.2 equiv.) as acidic catalyst and H₂O (2 mL) as the solvent. The reaction was performed at 250 °C for 3 h under 50 bar of N₂ pressure. Before the freeze drying step, NH₄Cl (s) was added until saturation. Pyrogallol was obtained in 45% NMR yield (0.45 mmol). ¹H NMR (400 MHz, DMSO-d₆): δ 8.50 (bs, 3H), 6.41 (t, *J ₌* 8.0 Hz, 1H), 6.24 (d, *J* = 8.0 Hz, 2H) ppm. ¹³C NMR (101 MHz, DMSO-d₆): δ 146.3 (C), 133.1 (C), 118.4 (CH), 107.1 (CH) ppm.

### Example 29. Synthesis of pyrogallol from dihydrosinapylalcohol (4-(3-hydroxypropyl)-2,6-dimethoxyphenol) [JBO-531]

This experiment was performed according to General procedure A. Dihydrosinapylalcohol (212 mg, 1.0 mmol) was used as the substrate, conc. H₂SO₄ (56 µL, 1 mmol, 1 equiv.) as acidic catalyst and H₂O (2 mL) as the solvent. The reaction was performed at 200 °C for 16 h under 50 bar of N₂ pressure. Pyrogallol was obtained in 28% NMR yield (0.28 mmol).

### Example 30. Synthesis of phenol from tyrosine [JBO-562]

This experiment was performed according to a modified General procedure A. L-tyrosine (181 mg, 1 mmol) was used as the substrate, conc. HCI (0.17 mL, 2 mmol, 2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆ and ¹H NMR was performed with suppression of the H₂O signal. Phenol was obtained in 27% NMR yield (0.27 mmol), whereas the starting material remained for 74% NMR yield (0.736 mmol).

### Example 31. Synthesis of catechol (benzene-1,2-diol) from 1-(3,4-dimethoxyphenyl)-2-(2-methoxyphenoxy)propan-1,3-diol [SA-143]

This experiment was performed according to a modified General procedure A. 1-(3,4-dimethoxyphenyl)-2-(2-methoxyphenoxy)propane-1,3-diol (67 mg, 0.2 mmol) was used as the substrate, 1 M HCI (aq., 0.11 mL, 0.11 mmol, 0.55 equiv.) as acidic catalyst and H₂O (0.89 mL) as solvent. The reaction was performed at 275 °C for 3 h under 100 bar of N₂ pressure. Catechol was obtained in 30% NMR yield.

### Example 32. Synthesis of catechol (benzene-1,2-diol) from 3-[(2R*,3S*)-2-(3,4-dimethoxyphenyl)-3-(hydroxymethyl)-7-methoxy-2,3-dihydro-1-benzofuran-5-yl]propan-1-ol[SA-148]

This experiment was performed according to a modified General procedure A. 3-[(2R*,3S*)-2-(3,4-dimethoxyphenyl)-3-(hydroxymethyl)-7-methoxy-2,3-dihydro-1-benzofuran-5-yl]propan-1-ol (75 mg, 0.2 mmol) was used as the substrate, 1 M HCI (aq., 0.11 mL, 0.11 mmol, 0.55 equiv.) as acidic catalyst and H₂O (0.89 mL) as solvent. The reaction was performed at 275 °C for 3 h under 100 bar of N₂ pressure. Catechol was obtained in 13% NMR yield.

### Example 33. Synthesis of catechol (benzene-1,2-diol) from 2-(2-methoxyphenoxy)-1-[(2R*,3S*)-2-(3,4-dimethoxyphenyl)-3-(hydroxymethyl)-7-methoxy-2,3-dihydro-1-benzofuran-5-yl]-propaan-1,3-diol [SA-146]

This experiment was performed according to a modified General procedure A. 2-(2-methoxyphenoxy)-1-[(2R*,3S*)-2-(3,4-dimethoxyphenyl)-3-(hydroxymethyl)-7-methoxy-2,3-dihydro-1-benzofuran-5-yl]-propaan-1,3-diol (103 mg, 0.2 mmol) was used as the substrate, 1 M HCI (aq., 0.11 mL, 0.11 mmol, 0.55 equiv.) as acidic catalyst and H₂O (0.89 mL) as solvent. The reaction was performed at 275 °C for 3 h under 100 bar of N₂ pressure. Catechol was obtained in 14% NMR yield.

### Example 34. Synthesis of catechol (benzene-1,2-diol) and pyrogallol (benzene-1,2,3-triol) from 2-(2,6-dimethoxyphenoxy)-1-[(2R*,3S*)-2-(3,4-dimethoxyphenyl)-3-(hydroxymethyl)-7-methoxy-2,3-dihydro-1-benzofuran-5-yl]-propaan-1,3-diol [SA-145]

This experiment was performed according to a modified General procedure A. 2-(2,6-dimethoxyphenoxy)-1-[(2*R**,3S*)-2-(3,4-dimethoxyphenyl)-3-(hydroxymethyl)-7-methoxy-2,3-dihydro-1-benzofuran-5-yl]-propaan-1,3-diol (109 mg, 0.2 mmol) was used as the substrate, 1 M HCI (aq., 0.11 mL, 0.11 mmol, 0.55 equiv.) as acidic catalyst and H₂O (0.89 mL) as solvent. The reaction was performed at 275 °C for 3 h under 100 bar of N₂ pressure. Catechol was obtained in 7% NMR yield and pyrogallol was obtained in 27% yield.

### Example 35. Synthesis of pyrogallol (benzene-1,2,3-triol) from 4,4'[(1S,3aR*4S,6aR*)-tetrahydro-1H,3H-furo[3,4-c]furan-1,4-diyl]bis(2,6-dimethoxyphenol) [SA-149]

This experiment was performed according to a modified General procedure A. 4,4'[(1S,3*aR**,4*S**,6aR*)-tetrahydro-1H,3H-furo[3,4-c]furan-1,4-diyl]bis(2,6-dimethoxyphenol) (84 mg, 0.2 mmol) was used as the substrate, 1 M HCI (aq., 0.11 mL, 0.11 mmol, 0.55 equiv.) as acidic catalyst and H₂O (0.89 mL) as solvent. The reaction was performed at 275 °C for 3 h under 100 bar of N₂ pressure. Pyrogallol was obtained in 8% yield.

### PART II: C-deacylation of side chains

### Example 36. Acid screening for the synthesis of catechol from 1-(3,4-dimethoxyphenyl)propan-1-one -0.2 equiv. HCI [JBO-763]

This experiment was performed according to a modified General procedure A. 1-(3,4-Dimethoxyphenyl)propan-1-one (194 mg, 1 mmol) was used as the substrate, conc. HCI (17 µL, 0.2 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆ and ¹H NMR analysis was performed with suppression of the H₂O signal. Catechol was obtained in 87% NMR yield (0.869 mmol), Propanoic acid was obtained in 72% NMR yield (0.719 mmol) and methanol in 60% (1.21 mmol).

### Example 37. Acid screening for the synthesis of catechol from 1-(3,4-dimethoxyphenyl)propan-1-one-0.2 equiv. H₂SO₄ [EB-604]

This experiment was performed according to a modified General procedure A. 1-(3,4-Dimethoxyphenyl)propan-1-one (194 mg, 1.00 mmol) was used as the substrate, conc. H₂SO₄ (11 µL, 0.20 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆ and ¹H NMR analysis was performed with suppression of the H₂O signal. Catechol was obtained in 89% NMR yield (0.892 mmol), Propanoic acid was obtained in 79% NMR yield (0.786 mmol) and methanol in 41% (0.823 mmol).

### Example 38. Acid screening for the synthesis of catechol from 1-(3,4-dimethoxyphenyl)propan-1-one - 0.2 equiv. methanesulfonic acid (MsOH) [EB-605]

This experiment was performed according to a modified General procedure A. 1-(3,4-Dimethoxyphenyl)propan-1-one (194 mg, 1.00 mmol) was used as the substrate, conc. MsOH (13 µL, 0.20 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆ and ¹H NMR analysis was performed with suppression of the H₂O signal. Catechol was obtained in 81% NMR yield (0.808 mmol), Propanoic acid was obtained in 73% NMR Yield (0.725 mmol) and methanol in 47% (0.944 mmol).

### Example 39. Acid screening for the synthesis of catechol from 1-(3,4-dimethoxyphenyl)propan-1-one - 0.2 equiv. oxalic acid [EB-606] - REFERENCE EXAMPLE

This experiment was performed according to a modified General procedure A. 1-(3,4-Dimethoxyphenyl)propan-1-one (194 mg, 1.00 mmol) was used as the substrate, oxalic acid (18 mg, 0.20 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆and ¹H NMR analysis was performed with suppression of the H₂O signal. No catechol was obtained.

### Example 40. Solvent screening for the synthesis of catechol from 1-(3,4-dimethoxyphenyl)propan-1-one - H₂O/MeOH (80:20) [EB-176] - REFERENCE EXAMPLE

This experiment was performed according to General procedure A. 1-(3,4-Dimethoxyphenyl)propan-1-one (194 mg, 1 mmol) was used as the substrate, conc. HCI (17 µL, 0.2 mmol, 0.2 equiv.) as acidic catalyst and H₂O (1.6 mL) and methanol (0.4 mL) as solvents. The reaction was performed at 250 °C for 3 h under 50 bar of N₂ pressure. No catechol was obtained.

### Example 41. Solvent screening for the synthesis of catechol from 1-(3,4-dimethoxyphenyl)propan-1-one - H₂O/CH₃CN (80:20) [EB-177] - REFERENCE EXAMPLE

This experiment was performed according to General procedure A. 1-(3,4-Dimethoxyphenyl)propan-1-one (194 mg, 1 mmol) was used as the substrate, conc. HCI (17 µL, 0.2 mmol, 0.2 equiv.) as acidic catalyst and H₂O (1.6 mL) and acetonitrile (0.4 mL) as solvents. The reaction was performed at 250 °C for 3 h under 50 bar of N₂ pressure. No catechol was obtained.

### Example 42. Temperature screening for the synthesis of catechol from 1-(3,4-dimethoxyphenyl)propan-1-one- 200 °C [EB-168] - REFERENCE EXAMPLE

This experiment was performed according to General procedure A. 1-(3,4-Dimethoxyphenyl)propan-1-one (194 mg, 1 mmol) was used as the substrate, conc. HCI (17 µL, 0.2 mmol, 0.2 equiv.) as acidic catalyst and H₂O (2 mL) as the solvent. The reaction was performed at 200 °C for 3 h under 50 bar of N₂ pressure. Only traces of catechol were obtained.

### Example 43. Synthesis of phenol from 1-(4-hydroxyphenyl)propan-1-one using 0.2 equiv. HCl [JBO-539]

This experiment was performed according to a modified General procedure A. 1-(4-Hydroxyphenyl)propan-1-one (150 mg, 1 mmol) was used as the substrate, conc. HCI (17 µL, 0.2 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆and ¹H NMR analysis was performed with suppression of the H₂O signal. Phenol was obtained in 35% NMR yield (0.354 mmol), Propanoic acid was obtained in 38% NMR yield (0.719 mmol) and the substrate was recovered for 60% (0.603 mmol).

### Example 44. Synthesis of phenol from 1-(4-hydroxyphenyl)propan-1-one using 0.2 equiv. H₂SO₄ [JBO-541]

This experiment was performed according to a modified General procedure A. 1-(4-Hydroxyphenyl)propan-1-one (150 mg, 1 mmol) was used as the substrate, conc. H₂SO₄ (11 µL, 0.2 mmol, 0.2 equiv.) as acidic catalyst and H₂O (2 mL) as the solvent. The reaction was performed at 250 °C for 3 h under 50 bar of N₂ pressure. Instead of freeze drying the aqueous phase, the reaction mixture was mixed with DMSO-d₆and ¹H NMR analysis was performed with suppression of the H₂O signal. Phenol was obtained in 32% NMR yield (0.324 mmol), Propanoic acid was obtained in 37% NMR Yyield (0.367 mmol) and the substrate was recovered for 69% (0.686 mmol).

### Example 45. Synthesis of catechol from veratraldehyde (3,4-dimethoxybenzaldehyde) [JBO-408]

This experiment was performed according to General procedure A. Veratraldehyde (166 mg, 1 mmol) was used as the substrate, conc. HCl (17 µL, 0.2 mmol, 0.2 equiv.) as acidic catalyst and H₂O (10 mL) as the solvent. The reaction was performed at 250 °C for 3 h under 50 bar of N₂ pressure. Catechol was obtained in 38% yield (based on NMR analysis).

### PART III: C-deacylation of side chains (combined with O-dealkylation) after functionalization of starting material

### Example 46. Acid screening for the synthesis of catechol from 2-methoxy-4-propylphenol - 0.2 equiv. HCl [EB-274]

This experiment was performed according to General procedure C. 2-Methoxy-4-propylphenol (166 mg, 1.00 mmol) was used as the substrate; dimethyl carbonate (1 mL) as the alkylation reagent and the solvent, and Cs₂CO₃ (65.2 mg, 0.200 mmol, 0.2 equiv.) as the base for alkylation step; sodium persulfate (476 mg, 2.00 mmol, 2 equiv.) as the oxidation reagent, sodium acetate (98.4 mg, 1.20 mmol, 1.2 equiv.) as the base and acetonitrile (8 mL) and H₂O (8 mL) as the solvent mixture for oxidation step; conc. HCI (17 µL, 0.20 mmol, 0.2 equiv.) as acidic catalyst and H₂O (2 mL) as the solvent for C-dealkylation and O-dealkylation of side chains. The reaction was performed at 250 °C for 3 h under 50 bar of N₂ pressure. Catechol was obtained in 44% NMR yield (0.441 mmol).

### Example 47. Acid screening for the synthesis of catechol from 2-methoxy-4-propylphenol - 0.2 equiv. H₂SO₄ [EB-275]

This experiment was performed according to General procedure C. 2-Methoxy-4-propylphenol (166 mg, 1.00 mmol) was used as the substrate; dimethyl carbonate (1 mL) as the alkylation reagent and the solvent, and Cs₂CO₃ (65.2 mg, 0.200 mmol, 0.2 equiv.) as the base for alkylation step; sodium persulfate (476 mg, 2.00 mmol, 2 equiv.) as the oxidation reagent, sodium acetate (98.4 mg, 1.20 mmol, 1.2 equiv.) as the base and acetonitrile (8 mL) and H₂O (8 mL) as the solvent mixture for oxidation step; conc. H₂SO₄ (11 µL, 0.20 mmol, 0.2 equiv.) as acidic catalyst and H₂O (2 mL) as the solvent for C-dealkylation and O-dealkylation of side chains. The reaction was performed at 250 °C for 3 h under 50 bar of N₂ pressure. Catechol was obtained in 27% NMR yield (0.273 mmol).

### Example 48. Synthesis of catechol from 2-methoxy-4-propylphenol - tBuOOH as oxidant

This experiment was performed according to General procedure C. 2-Methoxy-4-propylphenol (166 mg, 1.00 mmol) was used as the substrate; dimethyl carbonate (1 mL) as the alkylation reagent and the solvent, and Cs₂CO₃ (65.2 mg, 0.200 mmol, 0.2 equiv.) as the base for alkylation step; tBuOOH 70% in H₂O (412 mL, 3.00 mmol, 3 equiv.) as the oxidation reagent, FeCl₃.6H₂O (27.0 mg, 0.100 mmol, 0.1 equiv.) as the catalyst and pyridine (10 mL) as the solvent for oxidation step; conc. HCl (17 µL, 0.20 mmol, 0.2 equiv.) as acidic catalyst and H₂O (2 mL) as the solvent for C-dealkylation and O-dealkylation of side chains. The reaction is performed at 250 °C for 3 h under 50 bar of N₂ pressure.

### Example 49. Synthesis of catechol from 2-methoxy-4-propylphenol - O₂/NHPI as oxidants

This experiment was performed according to General procedure C. 2-Methoxy-4-propylphenol (166 mg, 1.00 mmol) was used as the substrate; dimethyl carbonate (1 mL) as the alkylation reagent and the solvent, and Cs₂CO₃ (65.2 mg, 0.200 mmol, 0.2 equiv.) as the base for alkylation step; O₂ (1 bar) and N-hydroxyphthalimide (32.6 mg, 0.200 mmol, 0.2 equiv.) as the oxidation reagents, Co(OAc)₂.4H₂O (2.5 mg, 0.010 mmol, 0.01 equiv.) as the catalyst and BuOAc (1 mL) as the solvent for oxidation step; conc. HCI (17 µL, 0.20 mmol, 0.2 equiv.) as acidic catalyst and H₂O (2 mL) as the solvent for C-dealkylation and O-dealkylation of side chains. The reaction is performed at 250 °C for 3 h under 50 bar of N₂ pressure.

### Example 50. Synthesis of catechol from 2-methoxy-4-propylphenol - DDQ

This experiment was performed according to General procedure C. 2-Methoxy-4-propylphenol (166 mg, 1.00 mmol) was used as the substrate; dimethyl carbonate (1 mL) as the alkylation reagent and the solvent, and Cs₂CO₃ (65.2 mg, 0.200 mmol, 0.2 equiv.) as the base for alkylation step; 2,3-dichloro-5,6-dicyano-1,4-benzoquinone (499 mg, 2.20 mmol, 2 equiv.) as the oxidation reagent, HCOOH (4.60 mg, 0.100 mmol, 0.1 equiv.) as the catalyst and dioxane (9 mL) and H₂O as the solvent mixture for oxidation step; conc. HCI (17 µL, 0.20 mmol, 0.2 equiv.) as acidic catalyst and H₂O (2 mL) as the solvent for C-dealkylation and *O-*dealkylation of side chains. The reaction is performed at 250 °C for 3 h under 50 bar of N₂ pressure.

### PART IV: O-dealkylation using zeolites as catalyst

### EXAMPLES NOT ACCORDING TO THE CLAIMED INVENTION

### Example 51. Zeolite screening for the O-demethylation of 4-propylguaiacol - Beta (CP814E); SiO₂/Al₂O₃ = 25; H form [JBO-794]

This experiment was performed according to a modified General procedure A. 4-Propylguaiacol (166 mg, 1 mmol) was used as the substrate, zeolite beta (Zeolyst, CP814E, **SiO₂/Al₂O₃** = 25, H form) (50 mg) as acidic catalyst and H₂O (2 mL) as the solvent. The reaction was performed at 250 °C for 3 h under 50 bar of N₂ pressure. The zeolite was removed by filtration, using acetone, prior to freeze drying. 4-Propylcatechol was obtained in 47% yield. ¹H-NMR (400 MHz, DMSO-d₆): δ 8.56 (bs, 2H), 6.61 (d, *J* = 7.9 Hz, 1H), 6.55 (d, *J* = 1.8 Hz, 1H), 6.41 (dd, *J* = 7.9, 1.8 Hz, 1H), 2.36 (t, *J* = 7.4 Hz, 2H), 1.50 (sextet, *J* = 7.4 Hz, 2H), 0.85 (t, *J* = 7.4 Hz, 3H) ppm. ¹³C-NMR (101 MHz, DMSO-d₆): δ 144.9 (C), 143.1 (C), 132.9 (C), 118.9 (CH), 115.7 (CH), 115.4 (CH), 36.7 (CH₂), 24.3 (CH₂), 13.6 (CH₃) ppm. HRMS (ESI) for C₉H₁₃O₂[M+H]⁺ calcd. 153.0916, found 153.0916.

### Example 52. Zeolite screening for the O-demethylation of 4-propylguaiacol - Beta (CP814C); SiO₂/Al₂O₃ = 38; H form [JBO-896]

This experiment was performed according to a modified General procedure A. 4-Propylguaiacol (166 mg, 1 mmol) was used as the substrate, zeolite beta (Zeolyst, CP814C, SiO₂/Al₂O₃ = 38, H form) (50 mg) as acidic catalyst and H₂O (2 mL) as the solvent. The reaction was performed at 250 °C for 3 h under 50 bar of N₂ pressure. The zeolite was removed by filtration, using acetone, prior to freeze drying. 4-Propylcatechol was obtained in 64% yield.

### Example 53. Variation of the zeolyte beta (CP814C) loading for the O-demethylation of 4-propylguaiacol[JBO-904]

This experiment was performed according to a modified General procedure A. 4-Propylguaiacol (166 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 3 h under 50 bar of N₂ pressure. The zeolite was removed by filtration, using acetone, prior to freeze drying. 4-Propylcatechol was obtained in 96% yield.

### Example 54. Variation of the temperature (275 °C) for the O-demethylation of 4-propylguaiacol [JBO-909]

This experiment was performed according to a modified General procedure A. 4-Propylguaiacol (166 mg, 1 mmol) was used as the substrate, zeolite beta (Zeolyst, CP814C, SiO₂/Al₂O₃ = 38, H form) (50 mg) as acidic catalyst and H₂O (2 mL) as the solvent. The reaction was performed at 275 °C for 3 h under 75 bar of N₂ pressure. The zeolite was removed by filtration, using acetone, prior to freeze drying. 4-Propylcatechol was obtained in 79% yield.

### Example 55. Variation of the temperature (275 °C) and zeolyte beta loading (CP814C) for the O-demethylation of 4-propylguaiacol [JBO-911]

This experiment was performed according to a modified General procedure A. 4-Propylguaiacol (166 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 275 °C for 3 h under 75 bar of N₂ pressure. The zeolite was removed by filtration, using acetone, prior to freeze drying. 4-Propylcatechol was isolated in 99% yield.

### Example 56. O-demethylation of 4-propylsyringol [JBO-925]

This experiment was performed according to a modified General procedure A. 4-Propylsyringol (196 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 275 °C for 3 h under 75 bar of N₂ pressure. The zeolite was removed by filtration, using acetone, prior to freeze drying. 5-Propylpyrogallol was obtained in 53% yield and the mono-demethylated intermediate in 16%.

### Example 57. Longer reaction time for the O-demethylation of 4-propylsyringol [JBO-942]

This experiment was performed according to a modified General procedure A. 4-Propylsyringol (196 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 275 °C for 16 h under 75 bar of N₂ pressure. The zeolite was removed by filtration, using acetone, prior to freeze drying. 5-Propylpyrogallol was obtained in 66% yield and the mono-demethylated intermediate in 13%.

### Example 58. Temperature screening for the O-demethylation of 4-propylsyringol [JBO-951]

This experiment was performed according to a modified General procedure A. 4-Propylsyringol (196 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 16 h under 50 bar of N₂ pressure. The zeolite was removed by filtration, using acetone, prior to freeze drying. 5-Propylpyrogallol was obtained in 55% yield and the mono-demethylated intermediate in 10%.

### Example 59. Work-up solvent screening for the O-demethylation of 4-propylsyringol [JBO-956]

This experiment was performed according to a modified General procedure A. 4-Propylsyringol (196 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 16 h under 50 bar of N₂ pressure. The zeolite was removed by filtration, using *n-*propanol, prior to freeze drying. 5-Propylpyrogallol was obtained in 72% yield and the mono-demethylated intermediate in 12%.

### Example 60. Zeolite screening for the O-demethylation of dihydroconiferylalcohol - Beta (CP814E); SiO₂/Al₂O₃ = 25; H form [JB0-900]

This experiment was performed according to a modified General procedure A. Dihydroconiferylalcohol (182 mg, 1 mmol) was used as the substrate, zeolite beta (Zeolyst, CP814E, SiO₂/Al₂O₃ = 25, H form) (50 mg) as acidic catalyst and H₂O (2 mL) as the solvent. The reaction was performed at 250 °C for 3 h under 50 bar of N₂ pressure. The zeolite was removed by filtration, using acetone, prior to freeze drying. Dihydrocaffeylalcohol was obtained in 26% yield. ¹H NMR (400 MHz, DMSO-d₆): δ 8.57 (bs, 2H), 6.61 (d, *J =* 8.0 Hz, 1H), 6.56 (d, *J* = 1.9 Hz, 1H), 6.41 (dd, *J* = 8.0, 1.9 Hz, 1H), 4.37 (bs, 1H), 3.40-3.34 (m, 2H), 2.41 (t, *J ₌* 7.3 Hz, 2H), 1.63 (quintet, *J ₌* 7.3 Hz, 2H) ppm. ¹³C NMR (101 MHz, DMSO-d₆): δ 144.9 (C), 143.0 (C), 133.0 (C), 118.8 (CH), 115.7 (CH), 115.4 (CH), 60.2 (CH₂), 34.6 (CH₂), 31.0 (CH₂) ppm.

### Example 61. Zeolite screening for the O-demethylation of dihydroconiferylalcohol - Beta (CP814C); SiO₂/Al₂O₃ = 38; H form [JBO-905]

This experiment was performed according to a modified General procedure A. Dihydroconiferylalcohol (182 mg, 1 mmol) was used as the substrate, zeolite beta (Zeolyst, CP814C, SiO₂/Al₂O₃ = 38, H form) (50 mg) as acidic catalyst and H₂O (2 mL) as the solvent. The reaction was performed at 250 °C for 3 h under 50 bar of N₂ pressure. The zeolite was removed by filtration, using acetone, prior to freeze drying. Dihydrocaffeylalcohol was obtained in 43% yield.

### Example 62. Zeolite loading screening for the O-demethylation of dihydroconiferylalcohol - Beta (CP814C); SiO₂/Al₂O₃ = 38; H form [JB0-906]

This experiment was performed according to a modified General procedure A. Dihydroconiferylalcohol (182 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 3 h under 50 bar of N₂ pressure. The zeolite was removed by filtration, using acetone, prior to freeze drying. Dihydrocaffeylalcohol was obtained in 70% yield.

### Example 63. Zeolite loading and reaction time screening for the O-demethylation of dihydroconiferylalcohol - Beta (CP814C); SiO₂/Al₂O₃ = 38; H form [JBO-931]

This experiment was performed according to a modified General procedure A. Dihydroconiferylalcohol (182 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 6 h under 50 bar of N₂ pressure. The zeolite was removed by filtration, using acetone, prior to freeze drying. Dihydrocaffeylalcohol was obtained in 61%.

### Example 64. Work-up solvent screening for the O-demethylation of dihydroconiferylalcohol - Beta (CP814C); SiO₂/Al₂O₃ = 38; H form [JBO-944]

This experiment was performed according to a modified General procedure A. Dihydroconiferylalcohol (182 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 6 h under 50 bar of N₂ pressure. The zeolite was removed by filtration, using n-propanol, prior to freeze drying. Dihydrocaffeylalcohol was obtained in 98% yield.

### Example 65. O-demethylation of dihydrosinapylalcohol [JBO-946]

This experiment was performed according to a modified General procedure A. Dihydrosinapylalcohol (212 mg, 1 mmol) was used as the substrate, zeolite beta (Zeolyst, CP814C, SiO₂/Al₂O₃ = 38, H form) (100 mg) as acidic catalyst and H₂O (2 mL) as the solvent. The reaction was performed at 250 °C for 6 h under 50 bar of N₂ pressure. The zeolite was removed by filtration, using n-propanol, prior to freeze drying. 5-(3-Hydroxypropyl)benzene-1,2,3-triol was obtained in 24% yield and the mono-demethylated intermediate in 17%.

### PART V: Alkylation and oxidation methods

As detailed already herein before, we herein also provide a method for preparing compounds suitable for use in the dealkylation and/or deacylation methods of the present invention. Such method is based on the functionalization (i.e. oxidation) of saturated non-funtionalized alkyl chains in the molecules. However, in order for the functionalization method to be carried out properly, first an alkylation (e.g. methylation) step of all free -OH groups need to be performed. While any suitable method for alkylation and oxidation may be used, we performed some several experiments to find suitable conditions for some particular methods as detailed below:
a) Alkylation method - tested conditions:

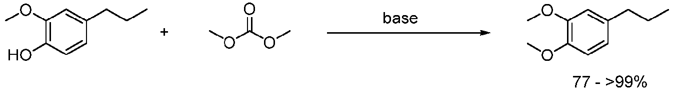

| **Entry** | **Base** | **Eq.** | **t (h)** | **T (°C)** | **Yield (%)** |
|---|---|---|---|---|---|
| **1** | K₂CO₃ | 0.2 | 15 | 160 | **97** |
| **2** | Cs₂CO₃ | 0.1 | 2 | 160 | **>99** |
| **3** | NEt₃ | 0.5 | 2 | 160 | **>99** |
| **4** | DBU | 0.5 | 2 | 160 | **>99** |
| **5** | CS₂CO₃ | 0.2 | 2 | 150 | **77** |

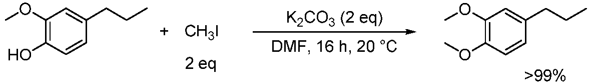

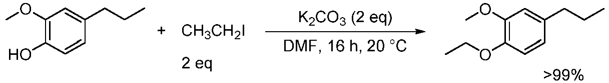

b) Oxidation method - tested conditions:

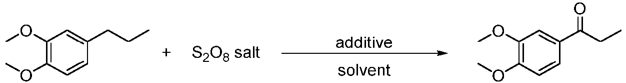

| **Entry** | **Oxidant** | **Eq.** | **Additive** | **Solvent** | **T (°C)** | **T (h)** | **Yield (%)** |
|---|---|---|---|---|---|---|---|
| **1** | K₂S₂O₈ | 2 | - | CH₃CN/H₂O | 77 | 3 | **36** |
| **2** | Na₂S₂O₈ | 2 | - | CH₃CN/H₂O | 77 | 3 | **36** |
| **3** | (NH₄)₂S₂O₈ | 2 | - | CH₃CN/H₂O | 77 | 3 | **17** |
| **4** | Na₂S₂O₈ | 2 | NaOAc (2) | CH₃CN/H₂O | 77 | 3 | **56** |
| **5** | Na₂S₂O₈ | 2 | NaOAc (2) | CH₃CN/H₂O | 77 | 1 | **12** |
| **6** | Na₂S₂O₈ | 2 | NaOAc.3H₂O (2) | CH₃CN/H₂O | 77 | 3 | **66** |
| **7** | Na₂S₂O₈ | 2 | HOAc | CH₃CN/H₂O | 77 | 3 | **48** |
| **8** | Na₂S₂O₈ | 2 | NaOAc (1.2) | CH₃CN/H₂O | 77 | 3 | **69** |
| **9** | Na₂S₂O₈ | 2 | NaOAc (2) | dioxane/ H₂O | 80 | 3 | **8** |
| **10** | Na₂S₂O₈ | 2 | NaOAc (2) | H₂O | 80 | 3 | **13** |
| **11** | Na₂S₂O₈ | 2 | NaOAc (2) | acetone/ H₂O | 80 | 3 | **16** |

Other oxidation methods as defined above, have also been tested, and provided the following results:

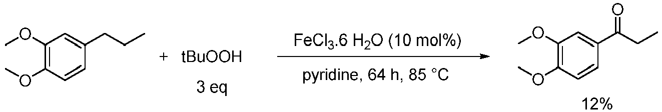

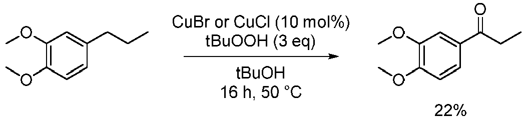

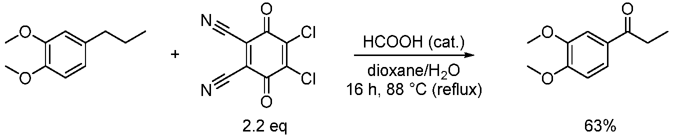

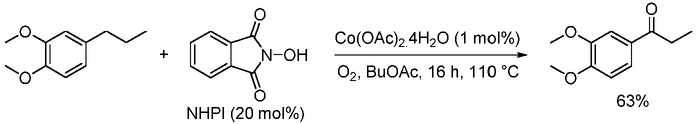

## Claims

1. A method for the dealkylation and/or deacylation of a compound of formula (I) 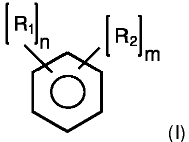 wherein:
each occurrence of R₁ is independently selected to be -OH, or -O-alkyl; wherein alkyl refers to a linear, branched or cyclic hydrocarbon group which may be saturated or contain one or more unsaturated bonds;
each occurrence of R₂ is independently selected from an alkyl group which may be linear, branched or cyclic; which may be saturated or containing one or more unsaturated bonds, or which may be an aromatic group; wherein said R₂ may optionally further contain one or more heteroatoms and/or one or more substituents;
n is 1-5;
m is 1-5; and the sum of n and m is maximally 6
said method comprising the steps of:
a) providing said compound of formula (I)
b) if in said compound of formula (I), said R₂ contains at least one alkyl group which does not have at least one functional moiety; then:
b1) alkylating any free -OH groups in said compound of step a); and
b2) oxidizing in said compound of step b1); said alkyl groups which do not have said at least one functional moiety;
wherein said at least one functional moiety is selected from the list consisting of: -OH, =O, a double bond or an amine;
c) preparing a reaction mixture by contacting said compound of step a), or where applicable step b), with an aqueous reaction mixture containing an acid having a pKa of maximum 3.0 or a Lewis acid, under an inert gas atmosphere;
wherein step c) is carried out at a temperature of at least 200 °C and a pressure of at least 20 bar;
d) obtaining from step c) a phenolic compound of formula (Ia) 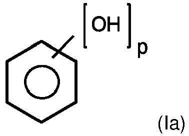
wherein p is equal to n.

2. The method according to claim 1; wherein at least one of said R₁ moieties is in ortho- or para- position with respect to at least one of said R₂ moieties.

3. The method according to any one of claims 1-2; wherein said acid is a mineral acid selected from the list consisting of: HCI, H₃PO₄, perchloric acid, chloric acid, HI, HBr, H₂SO₄, arene sulfonic acid, alkane sulfonic acid, nitric acid or a mixture of two or more of the afore-mentioned acids, or a Lewis acid such as a salt of metal such as Fe or Cu.

4. The method according to any one of claims 1-3; wherein the reaction mixture contains at least 0.05 equivalents of acid, preferably at least 0.1 equivalents with respect to the compound of formula I.

5. The method according to any one of claims 1-4; wherein the inert gas is selected from the list consisting of: N₂, CO₂, a noble gas, such as He, Ne, Ar, a gaseous alkane such as methane, or a mixture of two or more of the aforementioned gases.

6. The method according to any one of claims 1-5; wherein step c) is carried out at a temperature of at least 225 C, specifically at least 240°C, more specifically at least 250 °C; most specifically at least 275°C.

7. The method according to any one of claims 1-6; wherein step c) is carried out at a pressure of at least 30 bar, specifically at least 40 bar, more specifically at least 50 bar.

8. The method according to any one of claim 1 to 7; wherein the resulting end-product of formula (Ia) is catechol and said compound of formula (I) is selected from the list consisting of: caffeic acid, ferulic acid, dihydroconiferyl alcohol, propylguaiacol, and 1-(3,4-dimethoxyphenyl)propan-1-one.

9. The method according to any one of claims 1 to 7; wherein the resulting end-product of formula (Ia) is phenol and said compound of formula (I) is selected from the list consisting of: coumaric acid, tyrosine (L, D, or mixture of L/D isomers).

10. The method according to any one of claim 1 to 7; wherein the resulting end-product of formula (Ia) is pyrogallol and said compound of formula (I) is selected from the list consisting of: dihydrosinapyl alcohol, propylsyringol and 1-(3,4,5-trimethoxyphenyl)propan-1-one.

11. The method according to any one of claims 1 to 7; wherein the resulting end-product of formula (Ia) is phenol and said compound of formula (I) is selected from the list consisting of: hydrogenated cardanol and hydrogenated cashew nut shell liquid.

12. The method according to any one of claim 1 to 7; wherein the resulting end-product is a mixture of catechol and pyrogallol and said compound encompassing a moiety of formula (I) is a mixture selected from the list consisting of: dihydroconiferyl alcohol and dihydrosinapyl alcohol; propylguaiacol and propylsyringol; 1-(3,4-dimethoxyphenyl)propan-1-one and 1-(3,4,5-trimethoxyphenyl)propan-1-one.

## Patentansprüche

1. Verfahren für die Dealkylierung und/oder Deacetylierung einer Verbindung von Formel (I) 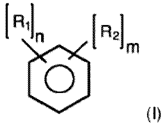 wobei:
jedes Vorkommen von R₁ unabhängig als -OH oder -O-Alkyl ausgewählt ist; wobei sich Alkyl auf eine lineare, verzweigte oder cyclische Kohlenwasserstoffgruppe bezieht, die gesättigt sein oder eine oder mehrere ungesättigte Bindungen enthalten kann;
jedes Vorkommen von R₂ aus einer Alkylgruppe unabhängig ausgewählt ist, die linear, verzweigt oder cyclisch sein kann; die gesättigt sein oder eine oder mehrere ungesättigte Bindungen enthalten kann oder eine aromatische Gruppe sein kann; wobei R₂ optional ferner ein oder mehrere Heteroatome und/oder einen oder mehrere Substituenten enthalten kann;
n 1-5 ist;
m 1-5 ist; und die Summe von n und m maximal 6 ist
das Verfahren umfassend die Schritte:
a) Bereitstellen der Verbindung von Formel (I)
b) falls in der Verbindung von Formel (I), das R₂ mindestens eine Alkylgruppe enthält, die nicht mindestens einen funktionellen Rest aufweist; dann:
b1) Alkylieren beliebiger freier -OH-Gruppen in der Verbindung aus Schritt a); und
b2) Oxidieren der Verbindung aus Schritt b1); die Alkylgruppen, die nicht mindestens einen funktionellen Rest aufweisen;
wobei die mindestens eine funktionelle Einheit aus der Liste ausgewählt ist, bestehend aus: -OH, =O, einer Doppelbindung oder einem Amin;
c) Herstellen eines Reaktionsgemisches durch Kontaktieren der Verbindung aus Schritt a) oder, sofern zutreffend, Schritt b) mit einem wässrigen Reaktionsgemisch, das eine Säure enthält, die einem pKa von maximal 3,0 oder eine Lewis-Säure aufweist, unter einer Inertgasatmosphäre;
wobei Schritt c) bei einer Temperatur von mindestens 200 °C und einem Druck von mindestens 20 bar ausgeführt wird;
d) Erhalten aus Schritt c) einer Phenolverbindung von Formel (la) 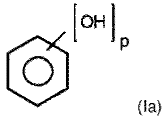 wobei p gleich n ist.

2. Verfahren nach Anspruch 1; wobei mindestens einer der R₁-Reste in ortho- oder para-Position hinsichtlich mindestens einem der R₂-Reste ist.

3. Verfahren nach einem der Ansprüche 1 bis 2; wobei die Säure eine Mineralsäure ist, die aus der Liste ausgewählt ist, bestehend aus: HCI, H₃PO₄, Perchlorsäure, Chlorsäure, HI, HBr, H₂SO₄, Arensulfonsäure, Alkansulfonsäure, Salpetersäure oder einem Gemisch aus zwei oder mehreren der zuvor genannten Säuren oder einer Lewis-Säure wie einem Salz eines Metalls wie Fe oder Cu.

4. Verfahren nach einem der Ansprüche 1 bis 3; wobei das Reaktionsgemisch mindestens 0,05 Äquivalente Säure, vorzugsweise mindestens 0,1 Äquivalente, hinsichtlich der Verbindung von Formel I enthält.

5. Verfahren nach einem der Ansprüche 1 bis 4; wobei das Inertgas aus der Liste ausgewählt wird, bestehend aus: N₂, CO₂, einem Edelgas wie He, Ne, Ar, einem gasförmigen Alkan wie Methan oder einem Gemisch aus zwei oder mehr der vorgenannten Gase.

6. Verfahren nach einem der Ansprüche 1 bis 5; wobei Schritt c) bei einer Temperatur von mindestens 225 °C, spezifisch mindestens 240 °C, spezifischer mindestens 250 °C; spezifischer mindestens 275 °C, ausgeführt wird.

7. Verfahren nach einem der Ansprüche 1 bis 6; wobei Schritt c) bei einem Druck von mindestens 30 bar, spezifisch mindestens 40 bar, spezifischer mindestens 50 bar ausgeführt wird.

8. Verfahren nach einem der Ansprüche 1 bis 7; wobei das resultierende Endprodukt von Formel (la) Brenzcatechin ist und die Verbindung von Formel (I) aus der Liste ausgewählt ist, bestehend aus: Kaffeesäure, Ferulasäure, Dihydroconiferylalkohol, Propylguajacol und 1-(3,4-Dimethoxyphenyl)propan-1-on.

9. Verfahren nach einem der Ansprüche 1 bis 7; wobei das resultierende Endprodukt von Formel (la) Phenol ist und die Verbindung von Formel (I) aus der Liste ausgewählt ist, bestehend aus: Cumarsäure, Tyrosin (L, D oder Gemisch von L/D-Isomeren).

10. Verfahren nach einem der Ansprüche 1 bis 7; wobei das resultierende Endprodukt von Formel (la) Pyrogallol ist und die Verbindung von Formel (I) aus der Liste ausgewählt ist, bestehend aus: Dihydrosinapylalkohol, Propylsyringol und 1-(3,4,5-Trimethoxyphenyl)propan-1-on.

11. Verfahren nach einem der Ansprüche 1 bis 7; wobei das resultierende Endprodukt von Formel (la) Phenol ist und die Verbindung von Formel (I) aus der Liste ausgewählt ist, bestehend aus: hydriertem Cardanol und hydrierter Cashewnussschalenflüssigkeit.

12. Verfahren nach einem der Ansprüche 1 bis 7; wobei das resultierende Endprodukt ein Gemisch aus Brenzcatechin und Pyrogallol ist und die Verbindung, die einen Rest von Formel (I) beinhaltet, ein Gemisch ist, das aus der Liste ausgewählt ist, bestehend aus: Dihydroconiferylalkohol und Dihydrosinapylalkohol; Propylguajacol und Propylsyringol; 1-(3,4-Dimethoxyphenyl)propan-1-on und 1-(3,4,5-Trimethoxyphenyl)propan-1-on.

## Revendications

1. Procédé destiné à la désalkylation et/ou à la désacylation d'un composé de formule (I) 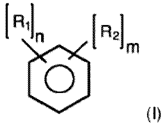 dans lequel :
chaque occurrence de R₁ est indépendamment choisie pour être -OH, ou -O-alkyle ; dans lequel alkyle fait référence à un groupe hydrocarboné linéaire, ramifié ou cyclique qui peut être saturé ou peut contenir une ou plusieurs liaisons insaturées ;
chaque occurrence de R₂ est indépendamment choisie parmi un groupe alkyle qui peut être linéaire, ramifié ou cyclique ; qui peut être saturé ou peut contenir une ou plusieurs liaisons insaturées, ou qui peut être un groupe aromatique ; dans lequel ledit R₂ peut facultativement contenir en outre un ou plusieurs hétéroatomes et/ou un ou plusieurs substituants ;
n va de 1 à 5 ;
m va de 1 à 5 ; et la somme de n et de m vaut au maximum 6
ledit procédé comprenant les étapes consistant à :
a) fournir ledit composé de formule (I)
b) si dans ledit composé de formule (I), ledit R₂ contient au moins un groupe alkyle qui n'a pas au moins un fragment fonctionnel ; alors :
b1) alkyler les groupes -OH libres éventuels dans ledit composé de l'étape a) ; et
b2) oxyder dans ledit composé de l'étape b1) ; lesdits groupes alkyle qui n'ont pas ledit au moins un fragment fonctionnel ; dans lequel ledit au moins un fragment fonctionnel est choisi dans la liste constituée par : -OH, ₌O, une double liaison ou une amine ;
c) préparer un mélange réactionnel par mise en contact dudit composé de l'étape a), ou le cas échéant de l'étape b), avec un mélange réactionnel aqueux contenant un acide ayant un pKa de maximum 3,0 ou un acide de Lewis, sous une atmosphère de gaz inerte ;
dans lequel l'étape c) est effectuée à une température d'au moins 200 °C et à une pression d'au moins 20 bar ;
d) obtenir de l'étape c) un composé phénolique de formule (la) 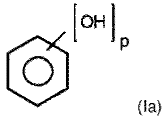
dans lequel p est égal à n.

2. Procédé selon la revendication 1 ; dans lequel au moins l'un desdits fragments R₁ est en position ortho ou para par rapport à au moins l'un desdits fragments R₂.

3. Procédé selon l'une quelconque des revendications 1 à 2 ; dans lequel ledit acide est un acide minéral choisi dans la liste constituée par : HCI, H₃PO₄, acide perchlorique, acide chlorique, HI, HBr, H₂SO₄, acide arène-sulfonique, acide alcane-sulfonique, acide nitrique ou un mélange de deux des acides susmentionnés ou plus, ou un acide de Lewis tel qu'un sel d'un métal tel que Fe ou Cu.

4. Procédé selon l'une quelconque des revendications 1 à 3 ; dans lequel le mélange réactionnel contient au moins 0,05 équivalent d'acide, de préférence au moins 0,1 équivalent par rapport au composé de formule I.

5. Procédé selon l'une quelconque des revendications 1 à 4 ; dans lequel le gaz inerte est choisi dans la liste constituée par : N₂, CO₂, un gaz noble, tel que He, Ne, Ar, un alcane gazeux tel que méthane, ou un mélange de deux des gaz susmentionnés ou plus.

6. Procédé selon l'une quelconque des revendications 1 à 5 ; dans lequel l'étape c) est effectuée à une température d'au moins 225 °C, spécifiquement au moins 240 °C, plus spécifiquement au moins 250 °C ; le plus spécifiquement au moins 275 °C.

7. Procédé selon l'une quelconque des revendications 1 à 6 ; dans lequel l'étape c) est effectuée à une pression d'au moins 30 bar, spécifiquement au moins 40 bar, plus spécifiquement au moins 50 bar.

8. Procédé selon l'une quelconque des revendications 1 à 7 ; dans lequel le produit final résultant de formule (la) est du catéchol et ledit composé de formule (I) est choisi dans la liste constituée par : acide caféique, acide férulique, alcool dihydroconiférylique, propylguaiacol et 1-(3,4-diméthoxyphényl)propan-1-one.

9. Procédé selon l'une quelconque des revendications 1 à 7 ; dans lequel le produit final résultant de formule (la) est du phénol et ledit composé de formule (I) est choisi dans la liste constituée par : acide coumarique, tyrosine (L, D ou mélange d'isomères L/D).

10. Procédé selon l'une quelconque des revendications 1 à 7 ; dans lequel le produit final résultant de formule (la) est du pyrogallol et ledit composé de formule (I) est choisi dans la liste constituée par : alcool dihydrosinapylique, propylsyringol et 1-(3,4,5-triméthoxyphényl)propane-1-one.

11. Procédé selon l'une quelconque des revendications 1 à 7 ; dans lequel le produit final résultant de formule (la) est du phénol et ledit composé de formule (I) est choisi dans la liste constituée par : cardanol hydrogéné et liquide de coque de noix de cajou hydrogéné.

12. Procédé selon l'une quelconque des revendications 1 à 7 ; dans lequel le produit final résultant est un mélange de catéchol et de pyrogallol et ledit composé englobant un fragment de formule (I) est un mélange choisi dans la liste constituée par : alcool dihydroconiférylique et alcool dihydrosinapylique ; propylguaiacol et propylsyringol ; 1-(3,4-diméthoxyphényl)propan-1-one et 1-(3,4,5-triméthoxyphényl)propan-1-one.
